# FASCICULE DE BREVET EUROPEEN

(11) **EP 1 345 945 B1**
(45) Date de publication et mention de la délivrance du brevet: **10.01.2007**
(21) Numéro de dépôt: 01995739.8
(22) Date de dépôt: 19.12.2001
(51) Int. Cl.: C07D 498/06, A61K 31/42

(54) **DERIVES DE STREPTOGRAMINES, LEUR PREPARATION ET LES COMPOSITIONS QUI LES CONTIENNENT**
STREPTOGRAMIN-DERIVATE, DEREN HERSTELLUNG UND DIE SIE ENHALTENDE ZUSAMMENSETZUNGEN
STREPTOGRAMIN DERIVATIVES, PREPARATION THEREOF AND COMPOSITIONS CONTAINING SAME

(30) Priorité: 21.12.2000 FR 0016803
(43) Date de publication de la demande: 24.09.2003
(73) Titulaire: Aventis Pharma S.A., 92160 Antony (FR)
(72) Inventeur: DESMAZEAU, Pascal, F-91250 TIGERY (FR); RONAN, Baptiste, F-92140 CLAMART (FR); BACQUE, Eric, F-91190 GIF SUR YVETTE (FR); BARRIERE, Jean-Claude, F-91440 BURES SUR YVETTE (FR)
(86) Numéro de dépôt international: PCT/FR2001/004061
(87) Numéro de publication internationale: WO 2002/050083

(56) Documents cités:
- EP-A- 0 135 410
- EP-A- 0 252 720
- WO-A-99/05165
- FR-A- 2 689 518
- GB-A- 2 206 879

## Description

La présente invention concerne des dérivés du groupe A des streptogramines de formule générale : 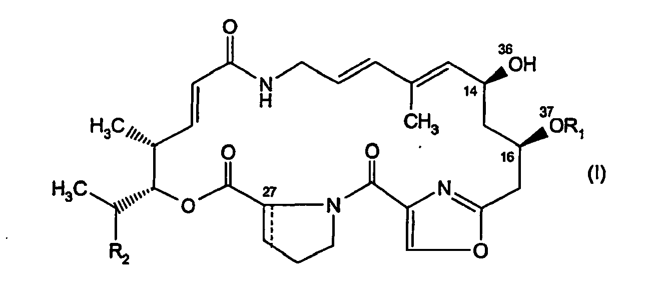 qui présentent une activité antibactérienne particulièrement intéressante.

Parmi les streptogramines connues, la pristinamycine (RP 7293), antibactérien d'origine naturelle produit par *Streptomyces pristinaespiralis* a été isolée pour la première fois en 1955. La pristinamycine commercialisée sous le nom de Pyostacine^{®} est constituée principalement de pristinamycine IIA associée à la pristinamycine IA.

Un autre antibactérien de la classe des streptogramines : la virginiamycine, a été isolé à partir de *Streptomyces virginiae,* ATCC 13161 [Antibiotics and Chemotherapy, 5, 632 (1955)]. La virginiamycine (Staphylomycine^{®}) est constituée principalement de facteur M₁ (VM1) associé au facteur S (VS).

F. Le Goffic et coll., Eur J. Med. Chem.-Chimica Therapeutica, 16(1), 69-72 (1981) ont décrit la préparation de dérivés dihydroxylés de la pristinamycine IIA

Dans la demande de brevet GB 2 206 879 ont été décrits des dérivés modifiés du groupe A des streptogramines de structure : 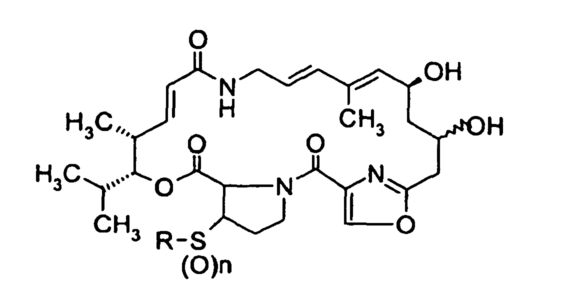 cependant ces dérivés ne manifestaient pas d'activité par voie orale.

Il a été maintenant trouvé, que les dérivés de streptogramine du groupe A de formule générale (I) dans laquelle :
- R₁ représente un radical alcoyle contenant 1 à 6 carbones en chaîne droite ou ramifiée ou alcényle ou alcynyle contenant 3 à 6 carbones en chaîne droite ou ramifiée, pouvant être mono ou polyfluorés, un radical cycloalcoyle contenant 3 à 6 atomes de carbone, phénylméthyle ou hétérocyclylméthyle dont la partie hétérocyclyle est aromatique et représente un cycle pyrrolyle, furyle, thiényle, imidazolyle ou pyridyle,
- R₂ représente un atome d'hydrogène ou un radical méthyle ou éthyle, et
- la liaison --- représente une liaison simple (stéréochimie 27R) ou une liaison double,
présentent une activité antibactérienne particulièrement intéressante, seuls ou associés à un dérivé de streptogramine du groupe B.

Selon l'invention, lorsque R₁ est alcoyle mono ou polysubstitué par un atome de fluor, le radical alcoyle contient de préférence 1 ou 2 atomes de carbone ; lorsque R₁ représente alcényle, il représente de préférence allyle et lorsqu'il représente alcynyle, il représente de préférence propargyle.

Les dérivés de streptogramine de formule générale (I) peuvent être préparés par action d'un dérivé de formule générale :

R₁-X (IIa)

pour lequel R₁ est défini comme précédemment et X représente un atome d'halogène, ou un radical méthylsulfonyloxy, p.toluènesulfonyloxy ou trifluorométhylsulfonyloxy, en présence d'un agent de transfert de phase, sur un dérivé dihydroxylé de streptogramine de formule générale : 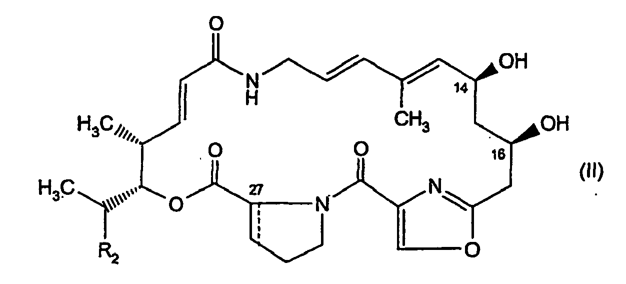 dans laquelle R₂ est défini comme précédemment, la liaison --- représente une liaison simple (stéréochimie 27R) ou une liaison double, et dont éventuellement la fonction hydroxy en position 14 et/ou la fonction amide en position 8 ont été préalablement protégées, suivie le cas échéant de l'élimination du/des radical(aux) protecteur(s).

De préférence, lorsque X est un atome d'halogène, on fait agir un dérivé de formule générale (IIa) pour lequel X est un atome de brome ou d'iode.

L'agent de transfert de phase est avantageusement choisi parmi les dérivés d'ammonium quaternaire (par exemple les sels de tétraalcoylammonium ou de trialcoylbenzylammonium comme le chlorure, le bromure ou le sulfate)

La réaction s'effectue généralement en milieu basique, par exemple en présence d'hydroxyde de sodium ou de potassium, ou en présence de carbonate de potassium ou de carbonate de césium, à une température comprise entre 10 et 60°C, en milieu hydro-organique, par exemple dans un hydrocarbure (toluène par exemple), un solvant halogéné (par exemple dichlorométhane) ou un ester (acétate d'éthyle notamment). De préférence on opère à environ 20°C. De préférence également on opère en présence d'un excès du dérivé de formule générale (IIa).

La protection et la déprotection du radical hydroxy en position 14 ou de l'amide en position 8 s'effectuent selon les méthodes habituelles qui n'affectent pas le reste de la molécule, notamment par application des méthodes décrites par T.W. Greene et P.G.M. Wuts, Protective Groups in Organic Synthesis (2^{ème} éd), A. Wiley - Interscience Publication (1991), ou par Mc Omie, Protective Groups in Organic Chemistry, Plenum Press (1973). Par exemple la protection du radical hydroxy s'effectue par un radical allyle qui est mis en place et éliminé par analogie avec les méthodes décrites ci-après dans les exemples. La protection de l'amide peut s'effectuer notamment par le radical t.butoxycarbonyle.

Lorsque la réaction conduit à un mélange des isomères 14- et 16-O-alcoylés, ces derniers peuvent être séparés selon les méthodes habituelles qui n'altèrent pas le reste de la molécule, notamment par chromatographie [Chromatographie Liquide Haute Performance (CLHP) sur phase normale ou inverse, sur phase chirale ou non ou par chromatographie flash] ou par cristallisation.

Selon l'invention, les dérivés de streptogramines de formule générale (I) peuvent être également préparés par désilylation puis 37-O-alcoylation au moyen d'un dérivé de formule générale (IIa), d'un dérivé silylé et 14-O-allylé de formule générale : 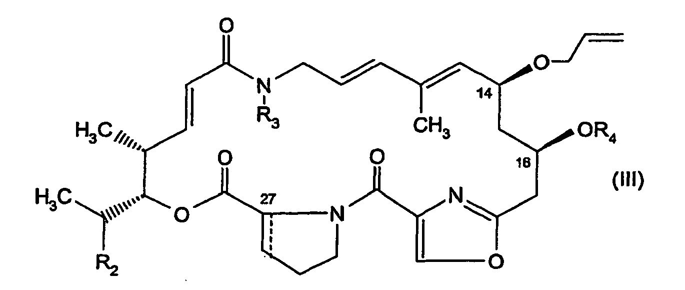 dans laquelle R₂ est défini comme précédemment, la liaison --- représente une liaison simple (stéréochimie 27R) ou une liaison double, et le radical R₃ représente un radical protecteur et R₄ représente un radical silylé, suivie de l'élimination du radical allyle et du radical protecteur R₃, selon les méthodes connues qui n'affectent pas le reste de la molécule.

Le radical protecteur R₃ est avantageusement t.butoxycarbonyle.
Le radical silylé R₄ peut être notamment choisi parmi trialcoylsilyle, dialcoylphénylsilyle ou alcoyldiphénylsilyle (par exemple t.butyldiphénylsilyle ou t.butyldiméthylsilyle).

La désilylation s'effectue selon les méthodes habituelles qui n'affectent pas le reste de la molécule. On opère notamment en présence d'une source d'ions fluorures comme le fluorure de tétra-n-butylammonium ou un complexe acide fluorhydrique-amine, l'amine pouvant être par exemple la triéthylamine ou la pyridine. La réaction s'effectue dans un solvant chloré (dichlorométhane par exemple) ou dans un éther (tétrahydrofuranne par exemple) à une température comprise entre 20 et 80°C.

La réaction de 37-O-alcoylation s'effectue par action d'un dérivé de formule générale (IIa) tel que défini précédemment, sur la streptogramine de formule générale (III) désilylée. De préférence on opère sous atmosphère inerte (par exemple sous azote ou sous argon) en milieu basique, par exemple en présence d'hydrure de sodium, d'hexaméthyldisylamidure alcalin (lithium, sodium, potassium) ou en présence d'un organolithien (n.butyllithium par exemple) ou encore en présence d'un amidure (par exemple le diisopropylamidure de lithium), dans un solvant inerte comme un amide (diméthylformamide par exemple) ou un éther (tétrahydrofurane par exemple), à une température comprise entre -20 et 60°C.

L'élimination du radical allyle s'effectue par exemple en présence d'un donneur de proton comme l'acide 4-méthylphénylsulfinique, en présence d'un catalyseur au palladium (palladium tetrakistriphénylphosphine par exemple), dans un solvant chloré (dichlorométhane par exemple), à une température comprise entre 0 et 60°C. Il est également possible d'opérer selon les méthodes décrites dans les références citées ci-avant. L'élimination du radical t.butoxycarbonyle s'effectue selon les méthodes qui n'affectent pas le reste de la molécule, notamment dans un solvant tel que le diméthylsulfoxyde, le diméthylformamide ou l'éther de diphényle, à une température comprise entre 130 et 170°C.

Le dérivé de streptogramine de formule générale (III) peut être préparé par 36-O-allylation, puis 37-O-silylation et protection de l'amide en 8 par un radical R₃.

L'allylation s'effectue selon les méthodes habituelles telles que citées dans les références ci-dessus. Notamment par action d'un halogénure d'allyle (bromure de préférence), ou d'un dérivé méthylsulfonyloxy, p.toluènesulfonyloxy ou trifluorométhylsulfonyloxy en présence d'une base comme un carbonate (de potassium ou de césium notamment), dans un solvant tel qu'une cétone (méthyléthylcétone par exemple), un nitrile (acétonitrile par exemple) ou un hydrocarbure (notamment toluène), à une température comprise entre 40 et 80°C, de préférence 70°C.

La silylation s'effectue selon les méthodes qui n'altèrent pas le reste de la molécule, notamment au moyen d'un halogénure (de préférence le chlorure) du radical silylé R₄. Par exemple on opère au moyen de chlorure de trialcoylsilyle, de dialcoylphénylsilyle ou d'alcoyldiphénylsilyle (par exemple le t.butyldiphénylsilyle ou le t.butyldiméthylsilyle) en opérant dans un solvant tel qu'un solvant chloré (dichlorométhane par exemple), un amide (diméthylformamide par exemple), un éther (tétrahydrofurane par exemple) ou un nitrile (acétonitrile par exemple) à une température comprise entre 0 et 60°C, de préférence on opère à température ambiante.

La mise en place du radical protecteur R₃ s'effectue selon les méthodes citées précédemment. Par exemple en présence d'un excès de dicarbonate de di-t.butyle, d'une base (triéthylamine, pyridine) et éventuellement d'un catalyseur comme la 4-diméthylaminopyridine, dans un solvant chloré (dichlorométhane) ou un éther (tétrahydrofuranne) à une température comprise entre 0 et 80°C.

Le dérivé dihydroxylé de streptogramine du groupe A de formule générale (II) peut être obtenu par réduction sélective de la composante de la pristinamycine naturelle de formule générale : 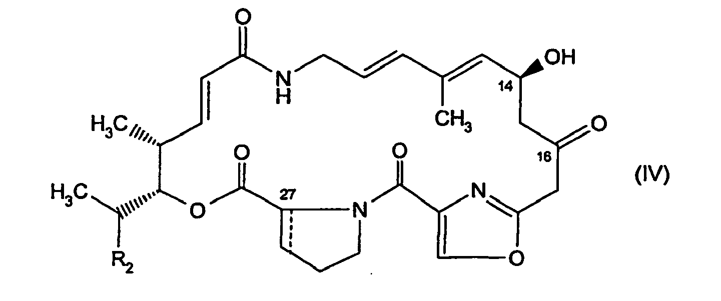 dans laquelle R₂ est défini comme ci-dessus et la liaison --- représente une liaison simple (stéréochimie 27R) ou une liaison double, suivie de la séparation de la forme épimère 16S.

La réduction s'effectue avantageusement en présence d'un agent réducteur tel qu'un borohydrure alcalin par exemple le borohydrure de sodium ou le triacétoxyborohydrure de sodium, dans un solvant organique choisi parmi les solvants chlorés (par exemple dichlorométhane, dichloréthane, chloroforme) le tétrahydrofurane, l'acide acétique et des alcools comme le méthanol, l'éthanol, ou le 2-propanol, à une température comprise entre -78 et 40°C

La séparation de la forme épimère 16R et de la forme épimère 16S s'effectue selon les méthodes habituelles ; par exemple la séparation des formes épimères peut s'effectuer par chromatographie, chromatographie flash, chromatographie liquide haute performance (CLHP), sur phase chirale ou non, ou chromatographie par partage centrifuge (CPC), à partir du mélange des épimères 16R et 16S. Ou par cristallisation.

Les dérivés de pristinamycine de formule générale (IV) correspondent respectivement à la pristinamycine IIA (PIIA), à la pristinamycine IIB (PIIB), à la pristinamycine IIC (PIIC), à la pristinamycine IID (PIID), à la pristinamycine IIF (PIIF) et à la pristinamycine IIG (PIIG) qui sont des composantes connues de la pristinamycine naturelle. Les composantes PIIF et PIIG ont été décrites dans le brevet européen EP 614910. La pristinamycine IIC (PIIC), et la pristinamycine IID (PIID) peuvent être obtenues comme décrit par J.C. Barrière et coll., Expert. Opin. Invest. Drugs, 3(2), 115-31(1994).

Les dérivés de pristinamycine de formule générale (III) sont des produits nouveaux.

La préparation et la séparation des composantes des streptogramines naturelles du groupe A [streptogramines de formule générale (IV)] est effectuée par fermentation et isolement des constituants à partir du moût de fermentation selon ou par analogie avec la méthode décrite par J. Preud'homme et coll., Bull. Soc. Chim. Fr., vol. 2, 585 (1968) ou dans le brevet européen EP 614910. Alternativement la préparation des composantes naturelles du groupe A peut être effectuée par fermentation spécifique, comme décrit dans la demande de brevet FR 2 689 518.

Les dérivés de streptogramine de formule générale (I) peuvent être purifiés le cas échéant par des méthodes physiques telles que la cristallisation, la chromatographie ou la CPC.

Les dérivés de streptogramine selon la présente invention présentent des propriétés antibactériennes et des propriétés synergisantes de l'activité antibactérienne des dérivés de streptogramine du groupe B. Ils sont particulièrement intéressants du fait de leur activité puissante seuls ou associés.

Lorsqu'ils sont associés avec une composante ou un dérivé du groupe B des streptogramines, ces derniers peuvent être choisis selon que l'on désire obtenir une forme administrable par voie orale ou parentérale, parmi les composantes naturelles : pristinamycine IA, pristinamycine IB, pristinamycine IC, pristinamycine ID, pristinamycine IE, pristinamycine IF, pristinamycine IG, virginiamycine S1, S3 ou S4, vernamycine B ou C, étamycine ou parmi des dérivés d'hémisynthèse tels que décrits dans les brevets ou demandes de brevet US 4618599, US 4798827, US 5326782, EP 772630 ou EP 770132, notamment des dérivés de streptogramines de formule générale : 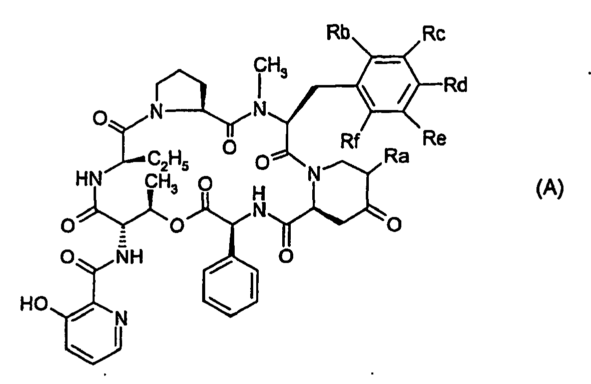 dans laquelle,
1. Rb, Rc, Re et Rf sont des atomes d'hydrogène, Rd est un atome d'hydrogène ou un radical diméthylamino, et Ra est un radical de structure -CH₂R'a pour lequel R'a est pyrrolidinyl-3thio, pipéridyl-3(ou -4)thio pouvant être substitués par alcoyle, alcoylthio substitué par 1 ou 2 hydroxysulfonyle, alcoylamino, dialcoylamino (lui même éventuellement substitué par mercapto ou dialcoylamino), ou substitué par 1
   ou 2 cycles pipérazine éventuellement substitué, morpholino, thiomorpholino, pipéridino, pyrrolidinyle-1, pipéridyle-2,-3 ou -4, ou pyrrolidinyle-2 ou -3 (pouvant être substitués par alcoyle), ou bien Ra est un radical de structure =CHR'a pour lequel R'a est pyrrolidinyl-3amino, pipéridyl-3(ou -4)amino, pyrrolidinyl-3oxy, pipéridyl-3(ou -4)oxy, pyrrolidinyl-3thio, pipéridyl-3(ou -4)thio pouvant être substitués par alcoyle, ou R'a est alcoylamino, alcoyloxy ou alcoylthio substitués par 1 ou 2 hydroxysulfonyle, alcoylamino, dialcoylamino (lui même éventuellement substitué par dialcoylamino), ou par trialcoylammonio, imidazolyl-4 ou -5, ou par 1 ou 2 cycles pipérazine éventuellement substitué, morpholino, thiomorpholino, pipéridino, pyrrolidinyle-1, pipéridyle-2,-3 ou -4, ou pyrrolidinyle-2 ou -3 (pouvant être substitués par alcoyle), ou
   Ra est un radical quinuclidinyl-3(ou -4)thiométhyle ou bien
2. Ra est un atome d'hydrogène et
   a) soit Rb, Re et Rf sont des atomes d'hydrogène, Rd est un radical -NHCH₃ ou -N(CH₃)₂ et Rc est un atome de chlore ou de brome, ou représente un radical alcényle contenant 3 à 5 atomes de carbone [si Rd est -N(CH₃)₂],
   b) soit Rb, Rd, Re et Rf représentent un atome d'hydrogène et Rc est un halogène, ou un radical aminomonoalkyle, aminodialkyle, alcoyloxy, trifluorométhyloxy, thioalcoyle,alcoyle en C₁ à C₃ ou trihalogénométhyle
   c) soit Rb, Rc, Re et Rf représentent un atome d'hydrogène et Rd est un halogène, ou un radical éthylamino, diéthylamino ou méthyléthylamino, alcoyloxy ou trifluorométhyloxy, thioalkyle, alcoyle en C₁ à C₆, aryle ou trihalogénométhyle
   d) soit Rb, Re et Rf représentent un atome d'hydrogène et Rc est halogène ou un radical aminomonoalkyle ou aminodialkyle, alcoyloxy ou trifluorométhyloxy, thioalkyle, alcoyle en C₁ à C₃, et Rd est halogène ou un radical amino, aminomonoalkyle ou aminodialkyle, alcoyloxy ou trifluorométhyloxy, thioalkyle, alcoyle en C₁ à C₆ ou trihalogénométhyle,
   e) soit Rc, Re et Rf représentent un atome d'hydrogène et Rb et Rd représentent un radical méthyle ;
ou encore parmi les dérivés d'hémisynthèse du groupe B des streptogramines de formule générale : 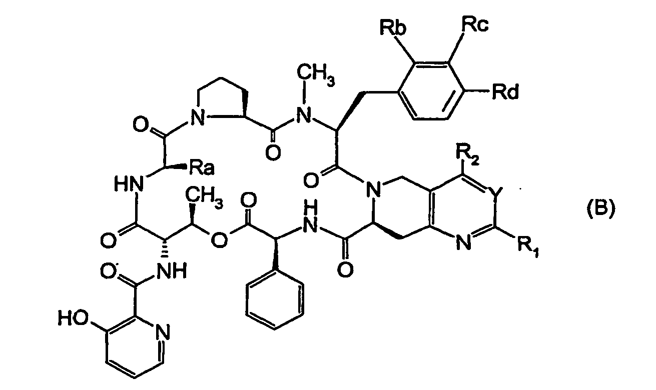 dans laquelle
Y est un atome d'azote ou un radical =CR₃-,
R₁ est un atome d'hydrogène, un radical alcoyle (1 à 8 carbones), alcényle (2 à 8 carbones), cycloalcoyle (3 à 8 carbones), hétérocyclyle saturé ou insaturé (3 à 8 chaînons), phényle, phényle substitué [par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino] ou un radical NR'R", R' et R" identiques ou différents pouvant être des atomes d'hydrogène ou des radicaux alcoyle (1 à 3 carbones), ou pouvant former ensemble avec l'atome d'azote auquel ils sont attachés, un hétérocycle de 3 à 8 chaînons contenant éventuellement un autre hétéroatome choisi parmi l'oxygène le soufre ou l'azote éventuellement substitué [par un radical alcoyle, alcényle (2 à 8 carbones), cycloalcoyle (3 à 6 carbones), hétérocyclyle saturé
ou insaturé (4 à 6 chaînons), benzyle, phényle ou phényle substitué tel que défini ci-dessus pour la définition de R₁]
ou bien lorsque Y est un radical =CR₃-, R₁ peut être aussi halogénométhyle, hydroxyméthyle, alcoyloxyméthyle, alcoylthiométhyle dont la partie alcoyle est éventuellement substituée par NR'R", alcoylsulfinylméthyle, alcoylsulfonylméthyle, acyloxyméthyle, benzoyloxyméthyle, cyclopropylaminométhyle ou -(CH₂)ₙNR'R" (n étant un entier de 1 à 4 et R' et R" étant définis comme ci-dessus), ou bien si R₃ est un atome d'hydrogène, R₁ peut être aussi formyle, carboxy, alcoyloxycarbonyle,
ou -CONR'R" pour lequel R' et R" sont définis comme ci-dessus,
ou bien lorsque Y est un atome d'azote, R₁ peut être aussi un radical -XR° pour lequel X est un atome d'oxygène ou de soufre, un radical sulfinyle ou sulfonyle, ou un radical NH et R° est un radical alcoyle (1 à 8 carbones), cycloalcoyle (3 à 6 carbones), hétérocyclyle saturé ou insaturé (3 à 8 chaînons), hétérocyclylméthyle (3 à 8 chaînons) dont la partie hétérocyclyle est attachée au radical méthyle par un atome de carbone, phényle, phényle substitué [par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino] ou un radical -(CH₂)ₙNR'R" pour lequel R' et R" sont définis comme ci-dessus et n est un entier de 2 à 4, ou bien, si X représente NH, R° peut aussi représenter l'atome d'hydrogène,
R₂ est un atome d'hydrogène ou un radical alcoyle (1 à 3 carbones),
R₃ est un atome d'hydrogène ou un radical alcoyle, carboxy, alcoyloxycarbonyle ou carbamoyle de structure -CO-NR'R" dans laquelle R' et R" sont définis comme précédemment,
Ra est un radical méthyle ou éthyle, et
Rb, Rc et Rd ont les définitions ci-après:
1) Rb et Rc sont des atomes d'hydrogène et Rd est un atome d'hydrogène ou un radical méthylamino ou diméthylamino,
2) Rb est un atome d'hydrogène, Rc est un atome d'hydrogène, de chlore ou de brome, ou représente un radical alcényle (3 à 5C), et Rd est un radical -NMe-R"' pour lequel R'" représente un radical alcoyle, hydroxyalcoyle (2 à 4C), ou alcényle (2 à 8C) éventuellement substitué par phényle, cycloalcoyl(3 à 6C)méthyle, benzyle, benzyle substitué [par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino], hétérocyclylméthyle ou hétérocyclyléthyle dont la partie hétérocyclyle est saturée ou insaturée et contient 5 à 6 chaînons et 1 ou 2 hétéroatomes choisis parmi le soufre, l'oxygène ou l'azote éventuellement substitué [par un radical alcoyle, alcényle (2 à 8 carbones), cycloalcoyle (3 à 6 carbones), hétérocyclyle saturé ou insaturé (4 à 6 chaînons), phényle, phényle substitué tel que défini ci-avant pour la définition de R₁ ou benzyle], ou bien R"' représente un radical cyanométhyle, ou -CH₂CORe pour lequel soit Re est -OR'e, R'e étant hydrogène, alcoyle (1 à 6 carbones), alcényle (2 à 6 carbones), benzyle ou hétérocyclylméthyle dont la partie hétérocyclyle contient 5 à 6 chaînons et 1 ou 2 hétéroatomes choisis parmi le soufre, l'oxygène ou l'azote soit Re est un radical alcoylamino, alcoyl méthyl amino, hétérocyclylamino ou hétérocyclyl méthyl amino dont la partie hétérocyclyle est saturée et contient 5 à 6 chaînons et 1 ou 2 hétéroatomes choisis parmi le soufre, l'oxygène ou l'azote éventuellement substitué par un radical alcoyle, benzyle ou alcoyloxycarbonyle,
3) Rb est un atome d'hydrogène, Rd est un radical -NHCH₃ ou -N(CH₃)₂ et Rc est un atome de chlore ou de brome, ou représente un radical alcényle (3 à 5C), [si Rd est -N(CH₃)₂],
4) Rb et Rd sont des atomes d'hydrogène et Rc est un atome d'halogène, ou un radical alcoylamino ou dialcoylamino, alcoyloxy, trifluorométhoxy, thioalcoyle, alcoyle (1 à 6C) ou trihalogénométhyle,
5) Rb et Rc sont des atomes d'hydrogène et Rd est un atome d'halogène, ou un radical éthylamino, diéthylamino ou méthyl éthyl amino, alcoyloxy ou trifluorométhoxy, alcoylthio, alcoylsulfinyle, alcoylsulfonyle, alcoyle (1 à 6C), phényle ou trihalogénométhyle,
6) Rb est un atome d'hydrogène et Rc est un atome d'halogène ou un radical alcoylamino ou dialcoylamino, alcoyloxy ou trifluorométhoxy, thioalcoyle, alcoyle (1 à 3C), et Rd est un atome d'halogène ou un radical amino, alcoylamino ou dialcoylamino, alcoyloxy ou trifluorométhoxy, thioalcoyle, alcoyle (1 à 6C) ou trihalogénométhyle,
7) Rc est un atome d'hydrogène et Rb et Rd représentent un radical méthyle, ainsi que leurs sels,
ou encore parmi les dérivés d'hémisynthèse du groupe B des streptogramines de formule générale : 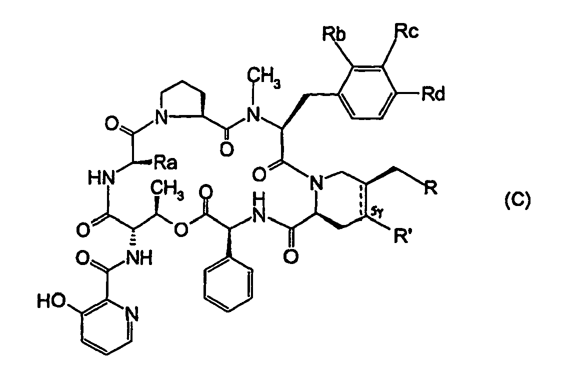 dans laquelle R représente un radical -NR₁R₂ ou -SR₃ [pour lequel R₁ et R₂ identiques
ou différents représentent H, alcoyle (1 à 8C) éventuellement substitué par OH, alcényle (3 à 8C), cycloalcoyle (3 à 8C), alcoyloxy (1 à 8C), dialcoylamino, phénylalcoyle éventuellement substitué [par un ou plusieurs halogène ou alcoyle, hydroxyalcoyle, alcoyloxy ou dialcoylamino], hétérocyclylalcoyle saturé ou insaturé (3 à 8 chaînons) contenant 1 ou plusieurs hétéroatomes choisis parmi N, S ou O, ou bien R₁ et R₂ forment ensemble avec l'atome d'azote, un hétérocycle mono ou polycyclique, saturé, partiellement saturé ou insaturé (3 à 12 chaînons), contenant éventuellement un autre hétéroatome choisi parmi N, S ou O, et éventuellement substitué [par un ou plusieurs OH, alcoyle, phényle éventuellement substitué par un atome d'halogène, phénylalcoyle, phénylalcényle (alcényle contenant 2 à 4C), hydroxyalcoyle, acyle, alcoyloxycarbonyle, ou hétérocyclyle ou hétérocyclylcarbonyle dont la partie hétérocyclyle est saturée ou insaturée (4 à 6 chaînons) et contient 1 ou plusieurs hétéroatomes choisis parmi N, S ou O,], R₃ est alcoyle-(1 à 8C) ou cycloalcoyle (3 à 8C) substitués par -NR₁R₂ pour lequel R₁ et R₂ identiques ou différents sont H ou alcoyle ou forment ensemble avec l'atome d'azote auquel ils sont attachés, un hétérocycle tel que défini ci-dessus, ou bien R₃ est hétérocyclyle ou hétérocyclylméthyle saturé ou insaturé (3 à 7 chaînons) et éventuellement un autre hétéroatome choisi parmi l'oxygène le soufre ou l'azote et éventuellement substitué par un radical alcoyle ; 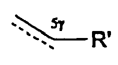 représente le reste d'un cycle insaturé non substitué en 5γ : 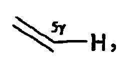 ou le reste d'un cycle saturé substitué en 5γ par un radical fluoro: 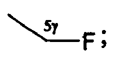 Ra est un radical Me ou Et, et Rb, Rc et Rd sont définis ci-après :
1) Rb et Rc sont H et Rd est H ou un radical MeNH ou NMe₂,
2) Rb est H, Rc est H, Cl ou Br, ou alcényle (3 à 5C), et Rd est -NMe-R"', R"' étant alcoyle, hydroxyalcoyle (2 à 4C), ou alcényle (2 à 8C), phénylalcényle, cycloalcoyl(3 à 6C)méthyle, benzyle, benzyle substitué, hétérocyclylméthyle, hétérocyclyléthyle, ou bien R"' est -CH₂CN, -CH₂COOH ou -CORe ou -CH₂CORe pour lesquels soit Re est -OR'e, soit Re est alcoylamino, alcoyl méthyl amino, hétérocyclylamino ou hétérocyclyl méthyl amino,
3) Rb est H, Rd est un radical -NHCH₃ ou -N(CH₃)₂ et Rc est Cl ou Br, ou alcényle (3 à 5C), [si Rd est -N(CH₃)₂],
4) Rb et Rd sont H et Rc est halogène, ou alcoylamino ou dialcoylamino, alcoyloxy, trifluorométhoxy, thioalcoyle, alcoyle (1 à 6C) ou trihalogénométhyle,
5) Rb et Rc sont H et Rd est halogène, ou éthylamino, diéthylamino ou méthyl éthyl amino, alcoyloxy ou trifluorométhoxy, alcoylthio, alcoylsulfinyle, alcoylsulfonyle, alcoyle (1 à 6C), phényle ou trihalogénométhyle,
6) Rb est H et Rc est halogène ou alcoylamino ou dialcoylamino, alcoyloxy ou trifluorométhoxy, thioalcoyle, alcoyle (1 à 3C), et Rd est halogène ou un radical amino, alcoylamino ou dialcoylamino, alcoyloxy ou trifluorométhoxy, thioalcoyle, alcoyle (1 à 6C) ou trihalogénométhyle
7) Rc est H et Rb et Rd sont CH₃
ainsi que ses sels lorsqu'ils existent

Il est entendu que les associations formées au moyen des dérivés selon l'invention et des streptogramines du groupe B entrent également dans le cadre de la présente invention.

Les dérivés du groupe B des streptogramines de structure (B) peuvent être préparés selon les méthodes décrites dans la demande internationale PCT/FR 99/00409. Les dérivés du groupe B des streptogramines de structure (C) peuvent être préparés selon les méthodes décrites dans la demande internationale PCT/FR 00/02146.

In vitro sur *Staphylococcus aureus IP8203,* les dérivés de streptogramine selon l'invention se sont montrés actifs à des concentrations comprises entre 0,06 et 32 µg/ml seuls ou associés à un dérivé du groupe B comme la pristinamycine IB; in vivo, ils synergisent l'activité antimicrobienne de la pristinamycine I_{B} sur les infections expérimentales de la souris à *Staphylococcus aureus IP8203* à des doses comprises entre 32 et 150 mg/kg par voie orale (DC₅₀).

Enfin, les produits selon l'invention sont particulièrement intéressants du fait de leur faible toxicité. Aucun des produits n'a manifesté de toxicité à des doses de 150 mg/kg sur *Staphylococcus aureus IP8203,* 2 fois par jour, par voie sous-cutanée ou par voie orale chez la souris.

Les dérivés de streptogramine de formule générale (I) pour lesquels :
- R₁ représente un radical alcoyle, alcényle ou alcynyle,
- R₂ représente un radical méthyle, et
- la liaison --- représente une liaison simple (stéréochimie 27R) ou une liaison double,
sont particulièrement intéressants.

Et parmi ces produits tout spécialement les produits décrits ci-après dans les exemples.

Parmi les dérivés du groupe A des streptogramines selon l'invention, on citera également les produits de formule générale (I) suivants :
. (16*R*)-16-Désoxo-16-trifluorométhoxy pristinamycine II_{B}
. (16*R*)-16-Désoxo-16-trifluorométhoxy pristinamycine II_{A}
. (16*R*)-16-Désoxo-16-difluorométhoxy pristinamycine II_{B}
. (16*R*)-16-Désoxo-16-difluorométhoxy pristinamycine II_{A}
. (16*R*)-16-Désoxo-16-fluorométhoxy pristinamycine II_{B}
. (16*R*)-16-Désoxo-16-fluorométhoxy pristinamycine II_{A}
. (16*R*)-16-Désoxo-16-cyclopropyloxy pristinamycine II_{B}
. (16*R*)-16-Désoxo-16-cyclopropyloxy pristinamycine II_{A}
. (16*R*)-16-Désoxo-16-cyclobutyloxy pristinamycine II_{B}
. (16*R*)-16-Désoxo-16-cyclobutyloxy pristinamycine II_{A}
. (16*R*)-16-Désoxo-16-isopropyloxy pristinamycine II_{B}
. (16*R*)-16-Désoxo-16-isopropyloxy pristinamycine II_{A}
. (16R)-16-Désoxo-16-(2-ffuoro-propèn-3-yloxy) pristinamycine II_{B}
. (16*R*)-16-Désoxo-16-(2-fluoro-propèn-3-yloxy) pristinamycine II_{A}.

Les exemples suivants illustrent la présente invention.

Dans les exemples qui suivent, la nomenclature 16-désoxo pristinamycine IIA (ou IIB) signifie le remplacement de la fonction cétone en position 16 par 2 atomes d'hydrogène. Au fur et à mesure de la chromatographie, toutes les fractions sont analysées par chromatographie en couche mince (CCM), sur plaques de silice Merck 60F254. Les fractions correspondants à un même tâche en CCM sont regroupées puis concentrées à sec, sous pression réduite (30°C; 2,7 kPa). Les résidus ainsi obtenus sont analysés par les techniques spectroscopiques habituelles (RMN; IR; MS) ce qui permet d'identifier le produit attendu.

### Exemple 1

### (16R)-16-Désoxo-16-méthoxy pristinamycine II_{B}

Une solution de 0,6 g de (16*R*)-8-N-tert-butyloxycarbonyl-16-désoxo-16-méthoxy pristinamycine II_{B} dans 15 cm³ de N,N-diméthylformamide est portée à ébullition pendant 2,5 heures. Le mélange réactionnel est versé dans 200 cm³ d'eau glacée puis la phase aqueuse est extraite par 3 fois 100 cm³ d'acétate d'éthyle. Les phases organiques sont rassemblées, lavées par 3 fois 200 cm³ d'une solution aqueuse saturée de chlorure de sodium, séchées sur sulfate de magnésium, filtrées puis concentrées sous pression réduite à sec (2,7 kPa) pour donner 0,65 g d'une huile jaune qui est purifiée par chromatographie-flash sur silice sur silice [éluant : dichlorométhane / méthanol / acétonitrile (90 / 5 / 5 en volumes)]. Après concentration des fractions contenant le produit attendu, on obtient 0,16 g d'une meringue jaune qui est recristallisée à chaud dans 3 cm³ d'acétonitrile pour donner 0,13 g de (16*R*)-16-désoxo-16-méthoxy pristinamycine II_{B}, sous forme d'une poudre blanche, fondant vers 124°C (déc.).

Spectre de R.M.N. ¹H (400 MHz, CDCl₃, δ en ppm) : 0,96 (d, J = 6,5 Hz : 3H) ; 1,00 (d, J = 6,5 Hz : 3H); 1,08 (d, J = 6,5 Hz : 3H); 1,70 (ddd, J = 14- 7 et 3 Hz : 1H); de 1,75 à 2,05 (mt : 5H); 1,82 (s : 3H) ; 1,89 (d, J = 3 Hz : 1H); 2,14 (mt : 1H); 2,75 (mt : 1H); 2,80 (dd, J= 16 et 8,5 Hz : 1H); 3,18 (dd, J=16 et 3,5 Hz : 1H); 3,45 (s : 3H); 3,50 (mt : 1H); 3,75 (mt : 1H); 3,95 (mt : 2H); 4,45 (mt : 1H); 4,70 (mt : 1H) ; de 4,75 à 4,85 (mt : 2H); 5,37 (d large, J = 9 Hz : 1H); 5,72 (ddd, J = 16-8 et 4,5 Hz : 1H); 5,81 (dd, J = 16,5 et 1,5 Hz : 1H); 6,07 (mt : 1H); 6,18 (d, J = 16 Hz : 1H) ; 6,51 (dd, J = 16,5 et 5 Hz : 1H) ; 8,10 (s : 1H).

La (16*R*)-8-N-tert-butyloxycarbonyl-16-désoxo-16-méthoxy pristinamycine II_{B} peut être préparée de la manière suivante :
A une solution de 0,41 g de p-toluènesulfinate de sodium dans 14 cm³ de dichlorométhane, on verse à 0°C, sous atmosphère d'argon, 2,4 cm³ d'acide chlorhydrique 1 N puis on laisse revenir à température ambiante en quinze minutes. Cette solution est séchée sur sulfate de magnésium, filtrée puis versée à 20°C, sous atmosphère d'argon, sur une solution de 0,93 g de (16*R*)-14-O-allyl-8-N-tert-butyloxycarbonyl-16-désoxo-16-méthoxy pristinamycine II_{B} et de 0,31 g de palladium tétrakistriphénylphosphine dans 18 cm³ de dichlorométhane. Après trente minutes d'agitation à température ambiante, le mélange réactionnel est versé sur 100 cm³ d'eau puis décanté. La phase organique est séchée sur sulfate de magnésium, filtrée puis concentrée sous pression réduite à sec (2,7 kPa) pour donner 1,66 g d'une huile jaune qui est purifiée par chromatographie-flash sur silice [éluant : dichlorométhane / acétonitrile/ méthanol (95 / 2,5 / 2,5 en volumes)]. Après concentration des fractions contenant le produit attendu, on obtient 0,63 g d'une meringue jaune qui est agitée dans 5 cm³ de pentane puis filtrée et séchée sous pression réduite (2,7 kPa) pour donner 0,6 g de (16*R*)-8-N-tert-butyloxycarbonyl-16-désoxo-16-méthoxy pristinamycine II_{B}, sous forme d'un solide jaune pâle.

Spectre de R.M.N. ¹H (400 MHz, CDCl₃, δ en ppm) : 0,96 (mt : 6H); 1,15 (d, J = 6,5 Hz : 3H); de 1,35 à 2,15 (mt : 7H) ; 1,57 (s : 9H); 1,81 (s : 3H); 2,41 (d, J = 2,5 Hz : 1H); 2,77 (mt : 1H); 2,87 (dd, J = 16 et 8 Hz : 1H); 3,21 (dd, J = 16 et 4 Hz : 1H); 3,46 (s : 3H); de 3,70 à 3,95 (mt : 3H); 4,12 (dd large, J = 14 et 4,5 Hz : 1H); 4,62 (dd, J = 14 et 9 Hz : 1H); 4,72 (mt : 1H); 4,81 (dd, J = 8 et 2,5 Hz : 1H); 4,89 (dd, J = 10 et 2 Hz : 1H); 5,52 (d large, J = 9 Hz : 1H); 5,67 (ddd, J = 16 - 9 et 4,5 Hz : 1H); 6,28 (d, J = 16 Hz : 1H); 6,94 (dd. J = 16 et 1,5 Hz : 1H) ; 7,04 (dd, J= 16 et 4 Hz: 1H); 8,14 (s: 1H).

La (16*R*)-14-O-allyl-8-N-tert-butyloxycarbonyl-16-désoxo-16-méthoxy pristinamycine II_{B} peut être préparée de la manière suivante :
A 0,4 g de (16*R*)-14-O-allyl-8-N-tert-butyloxycarbonyl-16-désoxo-16-hydroxy pristinamycine II_{B} en solution dans 5 cm³ de N,N-diméthylformamide, on ajoute à 25°C, sous atmosphère d'argon, 0,19 cm³ d'iodométhane et 0,043 g d'hydrure de sodium à 50 % dans la vaseline. Après 4 heures d'agitation, le mélange réactionnel est dilué avec 20 cm³ d'acétate d'éthyle puis versé sur 40 cm³ d'eau. La phase organique est décantée puis lavée par 40 cm³ d'une solution aqueuse saturée de chlorure de sodium, séchée sur sulfate de magnésium, filtrée puis concentrée sous pression réduite à sec (2,7 kPa) pour donner 0,31 g d'une huile jaune qui est purifiée par chromatographie-flash sur silice [éluant : dichlorométhane / acétonitrile / méthanol (95 / 2,5 / 2,5 en volumes)]. Après concentration des fractions contenant le produit attendu, on obtient 0,06 g de (16*R*)-14-O-allyl-8-N-tert-butyloxycarbonyl-16-désoxo-16-méthoxy pristinamycine II_{B}, sous forme d'un solide blanc.

Spectre de R.M.N. ¹H (400 MHz, CDCl₃, δ en ppm): 0,96 (d, J = 6,5 Hz : 6H); 1,15 (d, J = 6,5 Hz : 3H); de 1,40 à 2,15 (mt : 7H); 1,57 (s : 9H); 1,82 (s :3H); 2,78 (mt: 1H); 2,96 (dd, J = 16 et 6 Hz: 1H); 3,06 (dd, J = 16 et 4 Hz: 1H); 3,38 (s : 3H); de 3,70 à 3,85 (mt : 2H) ; 3,90 (mt : 2H) ; 3,98 (dd large, J = 12 et 5 Hz: 1H); 4,21 (dd, J=15 et 4 Hz: 1H); 4,37 (mt: 1H); 4,60(dd,J=15 et 8,5 Hz: 1H); 4,84(dd,J=8 et 2,5 Hz : 1H); 4,89 (dd, J = 10 et 2 Hz : 1H); 5,15 (dd, J = 10 et 1 Hz : 1H); 5,24 (dd, J = 18 et 1 Hz: 1H); 5,35 (d large, J = 9 Hz : 1H); 5,70 (ddd, J = 16 - 8,5 et 4 Hz : 1H); 5,89 (mt : 1H); 6,28 (d, J = 16 Hz : 1H); 6,98 (d, J = 16 Hz : 1H); 7,04 (dd, J= 16 et 4 Hz : 1H) ; 8,15 (s : 1H).

La (16*R*)-14-O-allyl-8-N-tert-butyloxycarbonyl-16-désoxo-16-hydroxy pristinamycine II_{B} peut être préparée de la manière suivante :
A 16,05 g de (16*R*)-14-O-allyl-16-(tert-butyldiméthylsilyloxy)-8-N-tert-butyloxycarbonyl-16-désoxo pristinamycine II_{B} en solution dans 80 cm³ de dichlorométhane, on ajoute 90 cm³ de trifluorohydrate de triéthylamine. Après 17 heures d'agitation à 40°C, le mélange réactionnel est dilué avec 100 cm³ de dichlorométhane puis versé sur 300 cm³ d'eau. Le pH de la phase aqueuse est ajusté à 8 par addition lente de bicarbonate de sodium. La phase organique est décantée puis séchée sur sulfate de sodium, filtrée et concentrée sous pression réduite à sec (2,7 kPa) pour donner 16,5 g d'une huile jaune qui est purifiée par chromatographie-flash sur silice [éluant : dichlorométhane / acétonitrile / méthanol (93 / 3,5 / 3,5 en volumes)]. Après concentration des fractions contenant le produit attendu, on obtient 10,23 g de (16R)-14-O-allyl-8-N-tert-butyloxycarbonyl-16-désoxo-16-hydroxy pristinamycine II_{B}, sous forme d'une meringue jaune.

Spectre de R.M.N. ¹H (400 MHz, CDCl₃, δ en ppm) : 0,96 (mt : 6H) ; 1,15 (d, J = 6,5 Hz : 3H) ; 1,43 (s : 9H); de 1,35 à 2,15 (mt : 6H); 1,78 (s : 3H) ; 2,27 (d mt, J = 15 Hz : 1H) ; 2,77 (mt : 1H) ; 2,83 (dd, J = 16 et 8 Hz : 1H) ; 3,05 (dd, J = 16 et 5,5 Hz : 1H) ; 3,55 (d, J = 1,5 Hz : 1H) ; de 3,70 à 3,95 (mt : 3H) ; 4,06 (dd large, J = 12 et 5 Hz : 1H) ; 4,15 (dd, J = 14,5 et 4 Hz : 1H) ; 4,31 (mt : 1H) ; 4,53 (t dédoublé, J = 9 et 4 Hz : 1H); 4,63 (dd, J = 14,5 et 9 Hz: 1H); 4,86 (dd, J = 8 et 2,5 Hz: 1H); 4,91 (dd, J = 10 et 2 Hz : 1H); 5,19 (dd, J = 10 et 1 Hz: 1H); 5,25 (dd, J = 17,5 et 1 Hz : 1H); 5,42 (d large, J = 9 Hz : 1H); 5,66 (ddd, J = 16 - 9 et 4 Hz : 1H); 5,91 (mt : 1H); 6,31 (d, J = 16 Hz : 1H); 6,92 (d, J = 16 Hz: 1H) ; 7,02 (dd, J = 16 et 4 Hz: 1H); 8,14 (s : 1H).

La (16*R*)-14-O-allyl-16-(tert-butyldiméthylsilyloxy)-8-N-tert-butyloxycarbonyl-16-désoxo pristinamycine II_{B} peut être préparée de la manière suivante :
A une solution de 1 g de (16*R*)-14-O-allyl-16-(tert-butyldiméthylsilyloxy)-16-désoxo pristinamycine II_{B} et de 3,4 g de di-tert-butyl-dicarbonate dans 30 cm³ de dichlorométhane, on ajoute 0,20 cm³ de triéthylamine et 0,12 g de 4-N,N-diméthylaminopyridine à 25°C. Le mélange réactionnel est agité 16 heures à température ambiante, dilué avec 30 cm³ de dichlorométhane puis versé sur 60 cm³ d'une solution aqueuse saturée de chlorure de sodium. La phase organique est décantée, séchée sur sulfate de magnésium et concentrée sous pression réduite à sec (2,7 kPa) pour donner 1,65 g d'une huile jaune qui est purifiée par chromatographie-flash sur silice [éluant : cyclohexane / acétate d'éthyle (60 / 40 en volumes)]. Après concentration des fractions contenant le produit attendu, on obtient 0,71 g de (16*R*)-14-O-allyl-16-(tert-butyldiméthylsilyloxy)-8-N-tert-butyloxycarbonyl-16-désoxo pristinamycine II_{B}, sous forme d'une huile épaisse jaune pâle.

Spectre de R.M.N. ¹H (400 MHz, CDCl₃, δ en ppm) : 0,10 (s : 6H) ; 0,92 (s : 9H); 0,96 (mt : 6H); 1,15 (d, J = 6,5 Hz : 3H); 1,43 (s : 9H); de 1,50 à 2,10 (mt : 7H); 1,83 (s : 3H); 2,77 (mt : 1H); 2,93 (dd, J = 14,5 et 5 Hz: 1H); 2,99 (dd, J = 14,5 et 3 Hz : 1H); 3,77 (dd large, J = 13 et 6 Hz : 1H); 3,90 (mt: 2H); 3,99 (dd large, J = 13 et 5 Hz : 1H); 4,12 (mt : 1H); de 4,25 à 4,35 (mt: 2H); 4,65 (mt : 1H); 4,83 (dd, J = 8 et 2,5 Hz : 1H); 4,89 (dd, J = 10 et 2 Hz : 1H); 5;14 (dd, J = 10 et 1,5 Hz : 1H); 5,25 (dd, J = 18 et 1,5 Hz : 1H); 5,39 (d large, J = 9 Hz: 1H); 5,68 (ddd, J = 16 - 9 et 4,5 Hz: 1H); 5,89 (mt : 1H; 6,32 (d, J = 16 Hz : 1H); 6,99 (d, J = 16 Hz : 1H); 7,06 (dd, J = 16 et 4 Hz: 1H) ; 8,16 (s : 1H).

La (16*R*)-14-O-allyl-16-(tert-butyldiméthylsilyloxy)-16-désoxo pristinamycine II_{B} peut être préparée de la manière suivante :
A une solution de 2 g de (16*R*)-14-O-allyl-16-hydroxy pristinamycine II_{B} et de 2,64 g de tert-butyldiméthylchorosilane dans 15 cm³ de dichlorométhane, on ajoute à 20°C, sous atmosphère d'argon, 3,06 cm³ de diisopropyléthylamine et de 0,43 g 4-N,N-diméthylaminopyridine. Après 17 heures d'agitation, le mélange réactionnel est dilué avec 50 cm³ de dichlorométhane puis versé sur 50 cm³ d'une solution aqueuse saturée de chlorure de sodium. La phase organique est décantée, séchée sur sulfate de magnésium, filtrée puis concentrée sous pression réduite à sec (2,7 kPa) pour donner un résidu qui est agité pendant 2 heures dans 30 cm³ d'éther diisopropylique. On obtient, après filtration et séchage sous pression réduite (2,7 kPa), 1,57 g de (16*R*)-14-O-allyl-16-(tert-butyldiméthylsilyloxy)-16-désoxo pristinamycine II_{B} sous forme d'une poudre blanche.

Spectre de R.M.N. ¹H (400 MHz, CDCl₃, δ en ppm) : 0,10 (s : 6H) ; 0,89 (s : 9H) ; 0,96 (d, J = 6,5 Hz : 3H) ; 1,00 (d, J = 6,5 Hz : 3H) ; 1,06 (d, J = 6,5 Hz : 3H) ; 1,60 (mt : 1H) ; de 1,75 à 2,05 (mt : 5H) ; 1,82 (s : 3H) ; 2,16 (mt : 1H) ; 2,75 (mt : 1H) ; 2,88 (dd, J = 16 et 7,5 Hz : 1H) ; 2,95 (dd, J = 16 et 4 Hz : 1H) ; 3,47 (mt : 1H) ; 3,79 (dd, J = 12 et 6 Hz : 1H) ; de 3,90 à 4,05 (mt : 3H) ; 4,13 (mt : 1H) ; 4,25 (mt : 1H) ; 4,50 (mt : 1H) ; de 4,70 à 4,85 (mt : 2H) ; 5,15 (dd, J = 10 et 1,5 Hz : 1H) ; 5,19 (d large, J = 9 Hz : 1H) ; 5,23 (dd, J = 17,5 et 1,5 Hz : 1H) ; 5,71 (ddd, J = 16 - 8,5 et 5 Hz : 1H) ; 5,81 (dd, J = 16,5 et 1,5 Hz : 1H) ; 5,88 (mt : 1H) ; 6,20 (d, J = 16 Hz : 1H) ; 6,28 (mt: 1H); 6,49 (dd, J =16,5 et 5 Hz : 1H); 8,05 (s : 1H).

La (16*R*)-14-O-allyl-16-désoxo-16-hydroxy pristinamycine II_{B} peut être préparée de la manière suivante :
A une solution de 20 g de (16*R*)-16-désoxo-16-hydroxy pristinamycine II_{B} dans 450 cm³ de 2-butanone, on ajoute à 20°C, 36,57 g de carbonate de potassium et 65,35 cm³ de bromure d'allyle. Le mélange réactionnel est chauffé au reflux pendant 76 heures. Après refroidissement à 20°C et filtration, le mélange réactionnel est concentré sous pression réduite (2,7 kPa). Le résidu est repris par 200 cm³ de dichlorométhane puis lavé successivement par 2 fois 100 cm³ d'eau et 300 cm³ d'une solution aqueuse saturée de chlorure de sodium. La phase organique est séchée sur sulfate de magnésium puis concentrée sous pression réduite à sec (2,7 kPa) pour donner 27,8 g d'une meringue jaune qui est purifiée par chromatographie-flash sur silice [éluant : dichlorométhane / acétonitrile / méthanol (94 / 3 / 3 en volumes)]. Après concentration des fractions contenant le produit attendu, on obtient 7,80 g de (16*R*)-14-O-allyl-16-désoxo-16-hydroxy pristinamycine II_{B} sous forme d'une meringue jaune.

Spectre de R.M.N. ¹H (400 MHz, CDCl₃, δ en ppm) : 0,96 (d, J = 6,5 Hz : 3H); 1,00 (d, J = 6,5 Hz : 3H); 1,10 (d, J = 6,5 Hz : 3H) ; de 1,70 à 2,05 (mt : 6H); 1,80 (s : 3H); 2,12 (mt : 1H); 2,74 (mt : 1H); 2,79 (dd, J = 16,5 et 5,5 Hz : 1H); 2,99 (dd, J = 16,5 et 7 Hz : 1H); 3,15 (d, J = 3 Hz : 1H) ; 3,42 (dd, J = 16-10 et 4Hz : 1H); de 3,80 à 3,95 (mt : 2H); de 3,95 à 4,10 (mt : 2H); 4,27 (mt : 1H); de 4,45 à 4,60 (mt : 2H) ; 4,78 (dd, J = 10 et 1,5 Hz : 1H); 4,83 (dd, J = 9 et 3,5 Hz : 1H); 5,18 (dd, J = 10,5 et 1,5 Hz : 1H); 5,25 (dd, J = 18 et 1,5 Hz : 1H); 5,34 (d large, J = 9 Hz : 1H); 5,65 (ddd, J = 16- 10 et 4,5 Hz : 1H); 5,79 (dd, J = 16 et 1,5 Hz : 1H); de 5,85 à 6,00 (mt : 2H) ; 6,22 (d large, J = 16 Hz : 1H) ; 6,51 (dd, J = 16 et 5 Hz : 1H) ; 8,15 (s : 1H).

La (16*R*)-16-désoxo-16-hydroxy pristinamycine II_{B} peut être préparée de la manière suivante :
Une suspension de 11,35 g de borohydrure de sodium dans 550 cm³ de dichlorométhane sont chauffés au reflux pendant 20 minutes. On ajoute alors 68,6 cm³ d'acide acétique goutte à goutte en environ 30 minutes puis une solution (préalablement séchée sur sulfate de sodium) de 52,75 g de pristinamycine II_{B} dans 230 cm³ de dichlorométhane en environ 45 minutes. Le mélange réactionnel est agité 4,5 heures au reflux puis 16 heures à 20°C. On ajoute alors au mélange réactionnel 500 cm³ de dichlorométhane et 1500 cm³ d'eau. La phase organique est décantée et la phase aqueuse est extraite par 500 cm³ de chlorure de méthylène. Les phases organiques sont réunies et le pH est ajusté à 8 par une addition lente de 1000 cm³ d'une solution aqueuse saturée de bicarbonate de sodium. La phase organique résultante est lavée successivement par 1000 cm³ d'eau et 1000 cm³ d'une solution aqueuse saturée de chlorure de sodium puis traitée au noir végétal 3S, séchée sur sulfate de sodium, filtrée sur Célite^{®} et concentrée sous pression réduite à sec (2,7 kPa) pour donner 50 g d'un solide jaune clair. A une solution du solide précédent dans 900 cm³ de chlorure de méthylène, on ajoute à 20°C, 378 cm³ d'une solution aqueuse d'hydroxyde d'ammonium 0,5 M. Après 16 heures d'agitation à 20°C, la phase organique est décantée, lavée par 1000 cm³ d'eau puis par 1000 cm³ d'une solution aqueuse saturée de chlorure de sodium, séchée sur sulfate de sodium, filtrée et concentrée sous pression réduite à sec (2,7 kPa) pour donner 46 g d'un solide jaune pâle qui est purifié par chromatographie-flash sur silice [éluant: gradient chlorure de méthylène / méthanol (98 / 2 et 97 / 3 en volumes)]. Après concentration des fractions contenant le produit attendu, on obtient 8,57 g de (16*R*)-16-désoxo-16-hydroxy pristinamycine II_{B}, sous forme d'une meringue blanc cassé fondant vers 140°C (déc.).

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,10 (d, J = 6,5 Hz : 3H) ; de 1,70 à 2,05 (mt : 6H) ; 1,81 (s : 3H) ; de 2,05 à 2,20 (mt : 2H) ; 2,76 (mt : 1H) ; 2,84 (dd, J = 16 et 5,5 Hz : 1H) ; 3,00 (dd, J = 16 et 7 Hz: 1H); 3,04 (d, J = 4 Hz: 1H); 3,45 (ddd, J = 16 - 9 et 4 Hz: 1H) ; 3,90 (mt : 1H) ; 4,04 (mt : 1H) ; 4,27 (mt : 1H) ; 4,48 (ddd, J = 16 - 9 et 4Hz : 1H) ; 4,80 (dd, J = 10 et 2 Hz : 1H) ; 4,84 (dd, J = 9 et 3,5 Hz : 1H) ; 4,88 (mt : 1H) ; 5,44 (d large, J = 9 Hz : 1H) ; 5,67 (ddd, J = 16 - 9 et 4 Hz : 1H) ; 5,80 (dd, J = 16 et 1,5 Hz : 111) ; 5,95 (dd, J = 9 et 4 Hz : 1H) ; 6,19 (d large, J = 16 Hz : 1H) ; 6,53 (dd, J = 16 et 5 Hz : 1H); 8,16 (s : 1H).

### Exemple 2

### (16R)-16-Désoxo-16-méthoxy pristinamycine II_{B}

A une solution de 8 g de (16*R*)-16-désoxo-16-hydroxy pristinamycine II_{B} (préparée comme décrit dans l'exemple 1) dans 40 cm³ de dichlorométhane et 40 cm³ d'eau, on ajoute à 25°C, 1,2 g d'hydroxyde de sodium, 0,50 g de bromure de tétra-n-butylammonium et 4,70 cm³ d'iodométhane. Le mélange réactionnel est agité pendant 24 heures à température ambiante puis décanté. La phase organique est lavée avec 3 fois 100 cm³ d'une solution aqueuse saturée de chlorure de sodium puis séchée sur sulfate de magnésium, filtrée et concentrée sous pression réduite (2,7 kPa) pour donner 10,7 g d'une meringue crème qui est purifiée par chromatographie-flash sur silice [éluant : dichlorométhane / acétonitrile / méthanol (95 / 2,5 / 2,5 en volumes)]. Après concentration des fractions contenant le produit attendu, on obtient 1,2 g d'une meringue jaune pâle qui est recristallisée à chaud dans 7 cm³ d'acétonitrile pour donner 1,15 g de (16*R*)-16-désoxo-16-méthoxy pristinamycine II_{B} sous forme de cristaux blanc fondant vers 125°C (déc.).

Spectre de R.M.N. ¹H (400 MHz, CDCl₃, δ en ppm) : 0,96 (d, J = 6,5 Hz : 3H); 1,00 (d, J = 6,5 Hz : 3H); 1,08 (d, J = 6,5 Hz : 3H) ; 1,70 (ddd, J =14 - 7 et 3 Hz : 1H); de 1,75 à 2,05 (mt : 5H); 1,82 (s: 3H); 1,92 (d, J = 4 Hz: 1H); 2,14 (mt : 1H); 2,75 (mt: 1H); 2,80 (dd, J = 16 et 8,5 Hz : 1H); 3,18 (dd, J = 16 et 4 Hz: 1H); 3,45 (s : 3H); 3,50 (mt : 1H); 3,75 (mt : 1H); 3,95 (mt : 2H) ; 4,45 (mt : 1H); 4,70 (mt : 1H) ; de 4,75 à 4,85 (mt : 2H); 5,36 (d large, J = 9 Hz : 1H); 5,72 (ddd, J= 16 - 8 et 4,5 Hz : 1H); 5,81 (dd, J =16,5 et 1,5 Hz : 1H); 6,06 (mt : 1H); 6,18 (d, J = 16 Hz : 1H) ; 6,51 (dd, J=16,5 et 5 Hz : 1H); 8,10 (s : 1H).

### Exemple 3

### (16R)-16-Allyloxy-16-désoxo pristinamycine II_{B}

A une solution de 7 g de (16*R*)-16-désoxo-16-hydroxy pristinamycine II_{B} (préparée comme décrit dans l'exemple 1) dans 50 cm³ de dichlorométhane et 50 cm³ d'eau, on ajoute à 25°C, 1,06 g d'hydroxyde de sodium, 0,40 g de bromure de tétra-n-butylammonium et 11,49 cm³ de bromure d'allyle. Le mélange réactionnel est agité pendant 24 heures à température ambiante puis décanté. La phase organique est lavée avec 2 fois 100 cm³ de solution aqueuse saturée de chlorure de sodium puis séchée sur sulfate de magnésium, filtrée et concentrée sous pression réduite (2,7 kPa) pour donner 7,15 g d'une meringue jaune qui est purifiée par chromatographie-flash sur silice [éluant : dichlorométhane / acétonitrile / méthanol (95 / 2,5 / 2,5 en volumes)].

Après concentration des fractions contenant le produit attendu, on obtient 1,2 g d'une meringue blanche qui est recristallisée à chaud dans 5 cm³ d'acétonitrile pour donner 0,68 g de (16*R*)-16-allyloxy-16-désoxo pristinamycine II_{B} sous forme de cristaux blanc fondant vers 114°C (déc.).

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,08 (d, J = 6,5 Hz : 3H) ; 1,73 (ddd, J = 15 - 7,5 et 3 Hz : 1H) ; de 1,75 à 2,05 (mt : 5H) ; 1,81 (s : 3H) ; 2,14 (mt : 1H) ; 2,74 (mt : 1H) ; 2,82 (dd, J = 16 et 8,5 Hz : 1H) ; 3,16 (dd, J = 16 et 3,5 Hz : 1H); 3,50 (ddd, J = 16 - 8,5 et 3,5 Hz : 1H) ; 3,91 (mt : 1H) ; 3,94 (mt : 2H) ; 4,00 (dd démultiplié, J = 12,5 et 5,5 Hz : 1H) ; 4,17 (dd démultiplié, J = 12,5 et 5,5 Hz : 1H) ; 4,45 (ddd, J = 16 - 9 et 5 Hz : 1H) ; 4,71 (mt : 1H) ; 4,76 (dd, J = 9 et 3,5 Hz :1H) ; 4,78 (dd, J = 10 et 1,5 Hz : 1H) ; 5,22 (dd, J = 10 et 1,5 Hz : 1H); 5,32 (dd, J= 17,5 et 1,5 Hz : 1H) ; 5,35 (d large, J = 9 Hz : 1H); 5,73 (ddd, J = 16 - 8,5 et 5 Hz : 1H); 5,81 (dd, J= 16 et 1,5 Hz : 1H); 5,96 (mt : 1H); 6,11 (mt : 1H); 6,17 (d large, J = 16 Hz : 1H); 6,51 (dd, J = 16 et 5 Hz : 1H) ; 8,08 (s : 1H).

### Exemple 4

### (16R)-16-Désoxo-16-(prop-2-ynyloxy) pristinamycine II_{B}

En opérant d'une manière analogue à celle décrite à l'exemple 2, mais à partir de 7 g de (16*R*)-16-désoxo-16-hydroxy pristinamycine II_{B} en solution dans 40 cm³ de dichlorométhane, on ajoute à 20°C, sous atmosphère d'argon, 1,7 g de bromure de tétra-n-butylammonium, 1,06 g d'hydroxyde de sodium, 40 cm³ d'eau et 5 cm³ de bromure de propargyle. Après 18 heures d'agitation et traitement, on obtient 7,23 g d'un solide orange qui est purifié par chromatographie-flash sur silice [éluant : dichlorométhane/ méthanol / acétonitrile (95 / 2,5 / 2,5 en volumes)]. Après concentration des fractions contenant le produit attendu, on obtient 1,58 g d'un solide jaune qui est recristallisé à chaud dans 40 cm³ d'acétonitrile. Après filtration sur verre fritté N°4, lavage des cristaux avec 40 cm³ d'acétonitrile, séchage sous pression réduite (2,7 kPa), on obtient 1,36 g de (16*R*)-16-désoxo-16-(prop-2-ynyloxy) pristinamycine II_{B}, sous forme de cristaux blancs fondant vers 112°C avec décomposition.

Spectre de R.M.N. ¹H (400 MHz, CDCl₃, δ en ppm) : 0,96 (d, J = 6,5 Hz : 3H) ; 1,01 (d, J = 6,5 Hz : 3H); 1,08 (d, J = 6,5 Hz : 3H); 1,75 (ddd, J = 16 - 7,5 et 3,5 Hz : 1H); de 1,75 à 2,05 (mt : 5H); 1,84 (s : 3H); 1,85 (d, J = 3,5 Hz : 1H); 2,15 (mt : 1H); 2,48 (t, J = 2,5 Hz : 1H); 2,75 (mt : 1H); 2,87 (dd, J = 16 et 9 Hz : 1H); 3,21 (dd, J = 16 et 3,5 Hz : 1H); 3,50 (ddd, J = 16 - 8 et 3,5 Hz : 1H); 3,94 (mt : 2H); 4,09 (mt : 1R) ; 4,25 (dd, J = 16 et 2,5 Hz : 1H) ; 4,31 (dd, J = 16 et 2,5 Hz : 1H) ; 4,46 (mt : 1H), de 4,65 à 4,80 (mt 2H) ; 4,78 (dd, J = 10 et 1,5 Hz : 1H) ; 5,36 (d large, J = 9 Hz : 1H) ; 5,74 (ddd, J = 16 - 8,5 et 5 Hz : 1H) ; 5,82 (dd, J = 16 et 1,5 Hz : 1H) ; de 6,10 à 6,25 (mt : 1H) ; 6,18 (d large, J = 16 Hz : 1H) ; 6,51 (dd, J =16 et 5,5 Hz : 1H) ; 8,10 (s : 1H).

### Exemple 5

### (16R)-16-Désoxo-16-méthoxy pristinamycine II_{A}

En opérant d'une manière analogue à celle décrite à l'exemple 2, mais à partir d'une solution de 8 g de (16*R*)-16-désoxo-16-hydroxy pristinamycine II_{A} dans 40 cm³ de dichlorométhane et 40 cm³ d'eau, on ajoute à 25°C, 1,2 g d'hydroxyde de sodium, 0,49 g de bromure de tétra-n-butylammonium et 4,7 cm³ d'iodure de méthyle. Après 96 heures d'agitation et traitement, on obtient, après purification par chromatographie-flash sur silice [éluant : dichlorométhane / acétonitrile / méthanol (96 / 2 / 2 en volumes)] et concentration des fractions contenant le produit attendu, 0,38 g d'une meringue qui est diluée dans 40 cm³ d'acétate d'éthyle puis lavée successivement avec 20 cm³ de solution d'acide chlorhydrique 0,1 N, 20 cm³ d'eau et 20 cm³ de solution aqueuse saturée de chlorure de sodium. La phase organique est séchée sur sulfate de magnésium, filtrée et concentrée sous pression réduite (2,7 kPa) pour donner 0,15 g de (16*R*)-16-désoxo-16-méthoxy pristinamycine II_{A} sous forme d'une poudre blanche, fondant vers 151°C avec décomposition.

Spectre de RM.N. ¹H (300 MHz, CDCl₃, δ en ppm) : 0,98 (d, J = 6,5 Hz : 3H) ; 1,00 (d, J = 6,5 Hz : 3H) ; 1,13 (d, J = 6,5 Hz : 3H) ; 1,50 (d, J = 3,5 Hz : 1H) ; 1,75 (d, J = 1 Hz : 3H) ; 1,88 (ddd, J = 16 - 10,5 et 3 Hz : 1H) ; de 1,95 à 2,10 (mt : 2H) ; de 2,60 à 2,90 (mt : 4H) ; 3,14 (dd, J = 14 et 3 Hz : 1H) ; 3,26 (mt : 1H) ; 3,44 (s : 3H) ; 3,87 (d très large, J = 17 Hz : 1H) ; de 4,15 à 4,45 (mt : 3H); 4,53 (mt : 1H) ; 4,86 (d large, J = 9Hz : 1H) ; 4,96 (dd, J = 10 et 2Hz: 1H); 5,66 (ddd, J = 16-5 et 3,5 Hz : 1H); 5,93 (d large, J = 16 Hz : 1H) ; 6,02 (dd, J = 16,5 et 1 Hz : 1H); 6,14 (t, J = 3 Hz : 1H); 6,62 (dd, J = 16,5 et 7,5 Hz : 1H); de 7,25 à 7,40 (mf : 1H); 7,89 (s : 1H).

La (16R)-16-hydroxy pristinamycine II_{A} peut être préparée selon F. Le Goffic; M-L. Capmau, J. Abbe, L. Charles et J. Montastier ; EUR. J. MED. - CHIMICA THERAPEUTICA; JANUARY - FEBRUARY, 1981 - 16, N°1, pp. 69 - 72.

La présente invention concerne également les compositions pharmaceutiques contenant au moins un dérivé de streptogramine selon l'invention, à l'état pur, associé à au moins un dérivé de streptogramine du groupe B, le cas échéant sous forme de sel, et/ou sous forme d'une association avec un ou plusieurs diluants ou adjuvants compatibles et pharmaceutiquement acceptables.

Les compositions selon l'invention peuvent être utilisées par voie orale, parentérale, topique, rectale ou en aérosols.

Comme compositions solides pour administration orale peuvent être utilisés des comprimés, des pilules, des gélules, des poudres ou des granulés. Dans ces compositions, le produit actif selon l'invention, généralement sous forme d'association est mélangé à un ou plusieurs diluants ou adjuvants inertes, tels que saccharose, lactose ou amidon. Ces compositions peuvent comprendre des substances autres que les diluants, par exemple un lubrifiant tel que le stéarate de magnésium ou un enrobage destiné à une libération contrôlée.

Comme compositions liquides pour administration orale, on peut utiliser des solutions pharmaceutiquement acceptables, des suspensions, des émulsions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles ou des émulsions. Comme solvant ou véhicule, on peut employer le propylèneglycol, un polyéthylèneglycol, des huiles végétales, en particulier l'huile d'olive, des esters organiques injectables, par exemple l'oléate d'éthyle. Ces compositions peuvent également contenir des adjuvants, en particulier des agents mouillants, isotonisants, émulsifiants, dispersants et stabilisants.

La stérilisation peut se faire de plusieurs façons, par exemple à l'aide d'un filtre bactériologique, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration topique peuvent être par exemple des crèmes, des pommades, des lotions ou des aérosols.

Les compositions par administration rectale sont les suppositoires ou les capsules rectales, qui contiennent outre le principe actif, des excipients tels que le beurre de cacao, des glycérides semi-synthétiques ou des polyéthylèneglycols.

Les compositions peuvent également être des aérosols. Pour l'usage sous forme d'aérosols liquides, les compositions peuvent être des solutions stériles stables ou des compositions solides dissoutes au moment de l'emploi dans de l'eau stérile apyrogène, dans du sérum ou tout autre véhicule pharmaceutiquement acceptable. Pour l'usage sous forme d'aérosols secs destinés à être directement inhalés, le principe actif est finement divisé et associé à un diluant ou véhicule solide hydrosoluble d'une granulométrie de 30 à 80 µm, par exemple le dextrane, le mannitol ou le lactose.

En thérapeutique humaine, les nouveaux dérivés de streptogramine selon l'invention sont particulièrement utiles dans le traitement des infections d'origine bactérienne. Les doses dépendent de l'effet recherché et de la durée du traitement. Le médecin déterminera la posologie qu'il estime la plus appropriée en fonction du traitement, en fonction de l'âge, du poids, du degré de l'infection et des autres facteurs propres au sujet à traiter. Généralement, les doses sont comprises entre 0,5 et 3 g de produit actif en 2 ou 3 administrations par jour, par voie orale ou parentérale pour un adulte.

L'exemple suivant illustre une composition selon l'invention.

### EXEMPLE

On prépare selon la technique habituelle des comprimés dosés à 250 mg de produit actif, ayant la composition suivante :
- (16*R*)-16-désoxo-16-méthoxy pristinamycine II_{B} 175 mg
- pristinamycine I_{B} 75 mg
- excipient : amidon, silice hydratée, dextrine, gélatine, stéarate de magnésium : qsp 500 mg

## Revendications

1. Un dérivé du groupe A des streptogramines de formule générale : 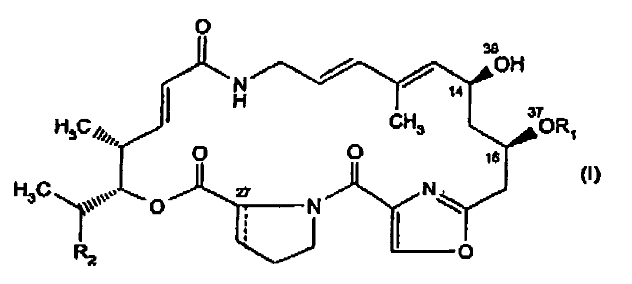 dans laquelle :
- R₁ représente un radical alcoyle contenant 1 à 6 carbones en chaîne droite ou ramifiée ou alcényle ou alcynyle contenant 3 à 6 carbones en chaîne droite ou ramifiée, pouvant être mono ou polyfluorés, un radical cycloalcoyle contenant 3 à 6 atomes de carbone, phénylméthyle ou hétérocyclylméthyle dont la partie hétérocyclyle est aromatique et représente un cycle pyrrolyle, furyle, thiényle, imidazolyle ou pyridyle,
- R₂ représente un atome d'hydrogène ou un radical méthyle ou éthyle, et
- la liaison --- représente une liaison simple (stéréochimie 27R) ou une liaison double.

2. Un dérivé de streptogramine selon la revendication 1, **caractérisé en ce que**
- R₁ représente un radical alcoyle, alcényle ou alcynyle,
- R₂ représente un radical méthyle, et
- la liaison --- représente une liaison simple (stéréochimie 27R) ou une liaison double.

3. Un procédé de préparation d'un dérivé du groupe A des streptogramines selon la revendication 1, **caractérisé en ce que** l'on fait agir un dérivé de formulé générale:
R₁-X (IIa)
pour lequel R₁ est défini comme dans la revendication 1 et X représente un atome d'halogène ou un radical méthylsulfonyloxy, p.toluènesulfonyloxy ou trifluorométhylsulfonyloxy, en présence d'un agent de transfert de phase, sur un dérivé dihydroxylé de streptogramine de formule générale : 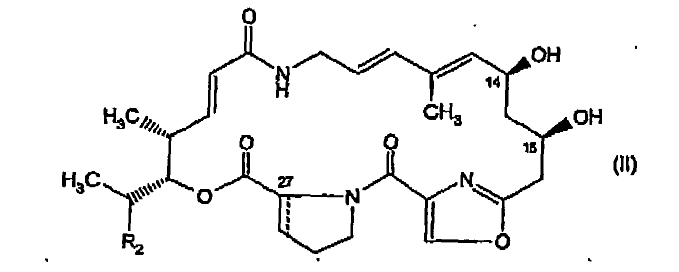 dans laquelle R₂ est défini comme dans la revendication 1, la liaison --- représente une liaison simple (stéréochimie 27R) ou une liaison double, et dont éventuellement la fonction hydroxy en position 14 et/ou la fonction amide en position 8 ont été préalablement protégées, suivie le cas échéant de l'élimination du/des radicaux protecteurs.

4. Un procédé de préparation d'un dérivé du groupe A des streptogramines selon la revendication 1, **caractérisé en ce que** l'on opère par désilylation puis 37-O-alcoylation d'un dérivé silylé et 14-Q-allylé de formule générale: 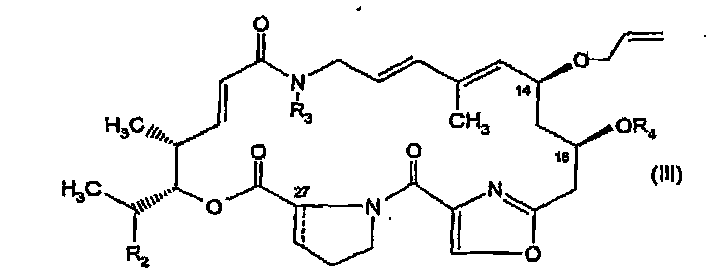 dans laquelle R₂ est défini comme dans la revendication 1, la liaison = représente une liaison simple (stéréochimie 27R) ou une liaison double, et le radical R₃ représente un radical protecteur et R₄, représente un radical silylé, au moyen d'un dérivé de formule générale (IIa) tel que défini dans la revendication 2, puis élimine le radical allyle et le radical protecteur R₃ selon les méthodes connues qui n'affectent pas le reste de la molécule.

5. Un procédé de préparation selon la revendication 4, **caractérisé en ce que** le radical R₃ est un radical t.butoxycarbonyle.

6. Un dérivé du groupe A des streptogramines **caractérisé en ce qu'**il répond à la formule générale : 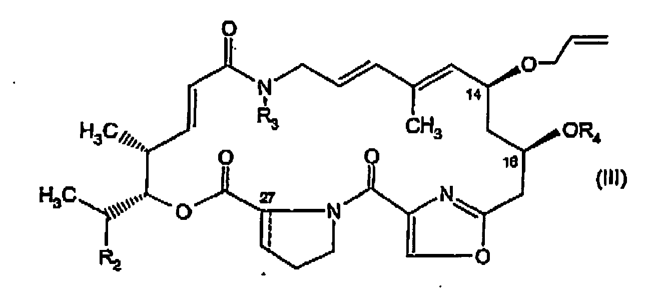 dans laquelle la liaison ---, R₂, R₃ et R₄ sont définis comme dans la revendication 4.

7. Composition pharmaceutique comprenant un dérivé de streptogramine du groupe A selon la revendication 1, à l'état pur ou sous forme d'association avec au moins un dérivé de streptogramine du groupe B, et/ou sous forme d'association avec un ou plusieurs diluants ou adjuvants compatibles et pharmaceutiquement acceptables.

8. Composition pharmaceutique selon la revendication 7, **caractérisée en ce que** le dérivé de la streptogramine du groupe B est choisi parmi les composantes naturelles: pristinamycine IA, pristinamycine IB, pristinamycine IC, pristinamycine ID, pristinamycine IE, pristinamycine IF, pristinamycine IG, virginiamycine S1, S3 au S4, vernamycine B ou C, étamycine ou parmi des dérivés d'hémisynthèse de formule générale: 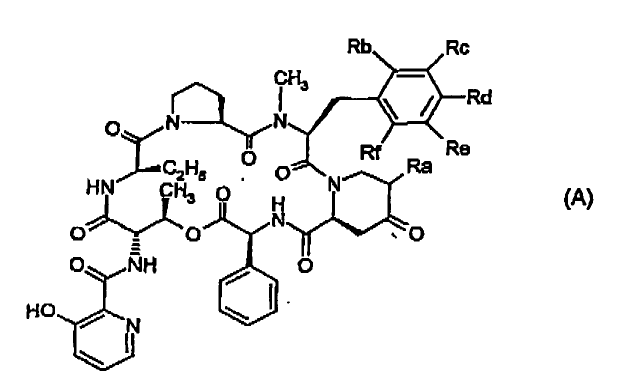 dans laquelle,
1) Rb, Rc, Re et Rf sont des atomes d'hydrogène, Rd est un atome d'hydrogène ou un radical diméthylamino, et Ra est un radical de structure -CH₂R'a pour lequel R'a est pyrrolidinyl-3thio, pipéridyl-3(ou -4)thio pouvant être substitués par alcoyle, alcoylthio substitué par 1 ou 2 hydroxysulfonyle, alcoylamino, dialcoylamino (lui même éventuellement substitué par mercapto ou dialcoylamino), ou substitué par 1 ou 2 cycles pipérazine éventuellement substitué, morpholino, thiomorpholino, pipéridino, pyrrolidinyle-1, pipéridyle-2,-3 ou -4, ou pyrrolidinyle-2 ou -3 (pouvant être substitués par alcoyle), ou bien Ra est un radical de structure =CHR'a pour lequel R'a est pyrrolidinyl-3am.ino, pipéridyl-3(ou -4)amino, pyrrolidinyl-3oxy, pipéridyl-3(ou -4)oxy, pyrrolidinyl-3thio, pipéridyl-3(ou -4)thio pouvant être substitués par alcoyle, ou R'a est alcoylamino, alcoyloxy ou alcoylthio substitués par 1 ou 2 hydroxysulfonyle, alcoylamino, dialcoylamino (lui même éventuellement substitué par dialcoylamino), ou par trialcoylammonio, imidazolyl-4 ou -5, ou par 1 ou 2 cycles pipérazine éventuellement substitué, morpholino, thiomorpholino, pipéridino, pyrrolidinyle-1, pipéridyle-2,-3 ou -4, ou pyrrolidinyle-2 ou -3 (pouvant être substitués par alcoyle), ou Ra est un radical quinuclidinyl-3(ou -4)thiométhyle ou bien
2) Ra est un atome d'hydrogène et
a) soit Rb, Re et Rf sont des atomes d'hydrogène, Rd est un radical -NHCH₃ ou -N(CH₃)₂ et Rc est un atome de chlore ou de brome, ou représente un radical alcényle contenant 3 à 5 atomes de carbone [si Rd est -N(CH₃)₂],
b) soit Rb, Rd, Re et Rf représentent un atome d'hydrogène et Rc est un halogène, ou un radical aminomonoalkyle, aminodialkyle, alcoyloxy, trifluorométhyloxy, thioalcoyle,alcoyle en C₁ à C₃ ou trihalogénométhyle
c) soit Rb, Rc, Re et Rf représentent un atome d'hydrogène et Rd est un halogène, ou un radical éthylamino, diéthylamino ou méthyléthylamino, alcoyloxy ou trifluorométhyloxy, thioalkyle, alcoyle en C₁ à C₆, aryle ou trihalogénométhyle
d) soit Rb, Re et Rf représentent un atome d'hydrogène et Rc est halogène ou un radical aminomonoalkyle ou aminodialkyle, alcoyloxy ou trifluorométhyloxy, thioalkyle, alcoyle en C₁ à C₃, et Rd est halogène ou un radical amino, aminomonoalkyle ou aminodialkyle, alcoyloxy ou trifluorométhyloxy, thioalkyle, alcoyle en C₁ à C₆ ou trihalogénométhyle,
e) soit Rc, Re et Rf représentent un atome d'hydrogène et Rb et Rd représentent un radical méthyle ;
ou encore parmi les dérivés d'hémisynthèse de formule générale : 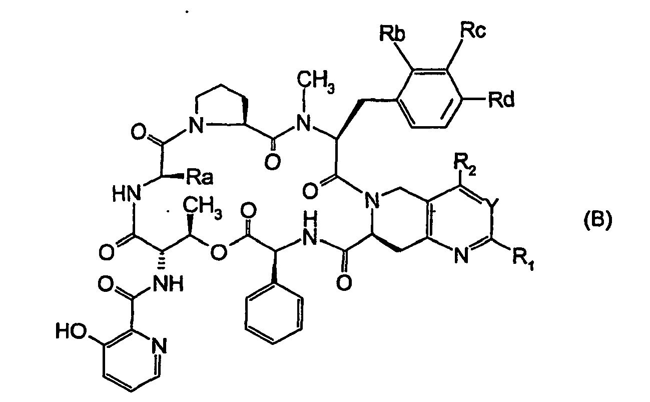 dans laquelle
Y est un atome d'azote ou un radical =CR₃-,
R₁ est un atome d'hydrogène, un radical alcoyle (1 à 8 carbones), alcényle (2 à 8 carbones), cycloalcoyle (3 à 8 carbones), hétérocyclyle saturé ou insaturé (3 à 8 chaînons), phényle, phényle substitué [par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino] ou un radical NR'R", R' et R" identiques ou différents pouvant être des atomes d'hydrogène ou des radicaux alcoyle (1 à 3 carbones), ou pouvant former ensemble avec l'atome d'azote auquel ils sont attachés, un hétérocycle de 3 à 8 chaînons contenant éventuellement un autre hétéroatome choisi parmi l'oxygène le soufre ou l'azote éventuellement substitué [par un radical alcoyle, alcényle (2 à 8 carbones), cycloalcoyle (3 à 6 carbones), hétérocyclyle saturé ou insaturé (4 à 6 chaînons), benzyle, phényle ou phényle substitué tel que défini ci-dessus pour la définition de R₁]
ou bien lorsque Y est un radical =CR₃-, R₁ peut être aussi halogénométhyle, hydroxyméthyle, alcoyloxyméthyle, alcoylthiométhyle dont la partie alcoyle est éventuellement substituée par NR'R", alcoylsulfinylméthyle, alcoylsulfonylméthyle, acyloxyméthyle, benzoyloxyméthyle, cyclopropylaminométhyle ou -(CH₂)ₙNR'R" (n étant un entier de 1 à 4 et R' et R" étant définis comme ci-dessus), ou bien si R₃ est un atome d'hydrogène, R₁ peut être aussi formyle, carboxy, alcoyloxycarbonyle, ou -CONR'R" pour lequel R' et R" sont définis comme ci-dessus,
ou bien lorsque Y est un atome d'azote, R₁ peut être aussi un radical -XR° pour lequel X est un atome d'oxygène ou de soufre, un radical sulfinyle ou sulfonyle,
ou un radical NH et R° est un radical alcoyle (1 à 8 carbones), cycloalcoyle (3 à 6 carbones), hétérocyclyle saturé ou insaturé (3 à 8 chaînons), hétérocyclylméthyle (3 à 8 chaînons) dont la partie hétérocyclyle est attachée au radical méthyle par un atome de carbone, phényle, phényle substitué [par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino] ou un radical -(CH₂)ₙNR'R" pour lequel R' et R" sont définis comme ci-dessus et n est un entier de 2 à 4, ou bien, si X représente NH, R° peut aussi représenter l'atome d'hydrogène,
R₂ est un atome d'hydrogène ou un radical alcoyle (1 à 3 carbones),
R₃ est un atome d'hydrogène ou un radical alcoyle, carboxy, alcoyloxycarbonyle ou carbamoyle de structure -CO-NR'R" dans laquelle R' et R" sont définis comme précédemment,
Ra est un radical méthyle ou éthyle, et
Rb, Rc et Rd ont les définitions ci-après :
1) Rb et Rc sont des atomes d'hydrogène et Rd est un atome d'hydrogène ou un radical méthylamino ou diméthylamino,
2) Rb est un atome d'hydrogène, Rc est un atome d'hydrogène, de chlore ou de brome, ou représente un radical alcényle (3 à 5C), et Rd est un radical -NMe-R"' pour lequel R"' représente un radical alcoyle, hydroxyalcoyle (2 à 4C), ou alcényle (2 à 8C) éventuellement substitué par phényle, cycloalcoyl(3 à 6C)méthyle, benzyle, benzyle substitué [par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino], hétérocyclylméthyle ou hétérocyclyléthyle dont la partie hétérocyclyle est saturée ou insaturée et contient 5 à 6 chaînons et 1 ou 2 hétéroatomes choisis parmi le soufre, l'oxygène ou l'azote éventuellement substitué [par un radical alcoyle, alcényle (2 à 8 carbones), cycloalcoyle (3 à 6 carbones), hétérocyclyle saturé ou insaturé (4 à 6 chaînons), phényle, phényle substitué tel que défini ci-avant pour la définition de R₁ ou benzyle], ou bien R"' représente un radical cyanométhyle, ou -CH₂CORe pour lequel soit Re est -OR'e, R'e étant hydrogène, alcoyle (1 à 6 carbones), alcényle (2 à 6 carbones), benzyle ou hétérocyclylméthyle dont la partie hétérocyclyle contient 5 à 6 chaînons et 1 ou 2 hétéroatomes choisis parmi le soufre, l'oxygène ou l'azote soit Re est un radical alcoylamino, alcoyl méthyl amino, hétérocyclylamino ou hétérocyclyl méthyl amino dont la partie hétérocyclyle est saturée et contient 5 à 6 chaînons et 1 ou 2 hétéroatomes choisis parmi le soufre, l'oxygène ou l'azote éventuellement substitué par un radical alcoyle, benzyle ou alcoyloxycarbonyle,
3) Rb est un atome d'hydrogène, Rd est un radical -NHCH₃ ou -N(CH₃)₂ et Rc est un atome de chlore ou de brome, ou représente un radical alcényle (3 à 5C), [si Rd est -N(CH₃)₂],
4) Rb et Rd sont des atomes d'hydrogène et Rc est un atome d'halogène, ou un radical alcoylamino ou dialcoylamino, alcoyloxy, trifluorométhoxy, thioalcoyle, alcoyle (1 à 6C) ou trihalogénométhyle,
5) Rb et Rc sont des atomes d'hydrogène et Rd est un atome d'halogène, ou un radical éthylamino, diéthylamino ou méthyl éthyl amino, alcoyloxy ou trifluorométhoxy, alcoylthio, alcoylsulfinyle, alcoylsulfonyle, alcoyle (1 à 6C), phényle ou trihalogénométhyle,
6) Rb est un atome d'hydrogène et Rc est un atome d'halogène ou un radical alcoylamino ou dialcoylamino, alcoyloxy ou trifluorométhoxy, thioalcoyle, alcoyle (1 à 3C), et Rd est un atome d'halogène ou un radical amino, alcoylamino ou dialcoylamino, alcoyloxy ou trifluorométhoxy, thioalcoyle, alcoyle (1 à 6C) ou trihalogénométhyle,
7) Rc est un atome d'hydrogène et Rb et Rd représentent un radical méthyle, ainsi que leurs sels,
ou encore parmi les dérivés d'hémisynthèse de formule générale : 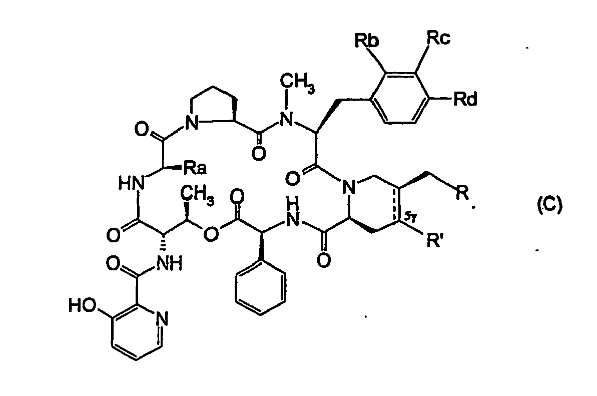 dans laquelle R représente un radical -NR₁R₂ ou -SR₃ [pour lequel R₁ et R₂ identiques ou différents représentent H, alcoyle (1 à 8C) éventuellement substitué par OH, alcényle (3 à 8C), cycloalcoyle (3 à 8C), alcoyloxy (1 à 8C), dialcoylamino, phénylalcoyle éventuellement substitué [par un ou plusieurs halogène ou alcoyle, hydroxyalcoyle, alcoyloxy ou dialcoylamino], hétérocyclylalcoyle saturé ou insaturé (3 à 8 chaînons) contenant 1 ou plusieurs hétéroatomes choisis parmi N, S ou O, ou bien R₁ et R₂ forment ensemble avec l'atome d'azote, un hétérocycle mono ou polycyclique, saturé, partiellement saturé ou insaturé (3 à 12 chaînons), contenant éventuellement un autre hétéroatome choisi parmi N, S ou O, et éventuellement substitué [par un ou plusieurs OH, alcoyle, phényle éventuellement substitué par un atome d'halogène, phénylalcoyle, phénylalcényle (alcényle contenant 2 à 4C), hydroxyalcoyle, acyle, alcoyloxycarbonyle, ou hétérocyclyle ou hétérocyclylcarbonyle dont la partie hétérocyclyle est saturée ou insaturée (4 à 6 chaînons) et contient 1 ou plusieurs hétéroatomes choisis parmi N, S ou O,], R₃ est alcoyle (1 à 8C) ou cycloalcoyle (3 à 8C) substitués par NR₁R₂ pour lequel R₁ et R₂ identiques ou différents sont H ou alcoyle ou forment ensemble avec l'atome d'azote auquel ils sont attachés, un hétérocycle tel que défini ci-dessus,
ou bien R₃ est hétérocyclyle ou hétérocyclylméthyle saturé ou insaturé (3 à 7 chaînons) et éventuellement un autre hétéroatome choisi parmi l'oxygène le soufre ou l'azote et éventuellement substitué par un radical alcoyle ; 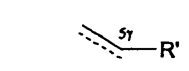 représente le reste d'un cycle insaturé non substitué en 5γ : 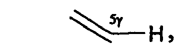 ou le reste d'un cycle saturé substitué en 5γ par un radical fluoro : 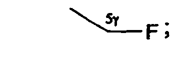 Ra est un radical Me ou Et, et Rb, Rc et Rd sont définis ci-après :
1) Rb et Rc sont H et Rd est H ou un radical MeNH ou NMe₂,
2) Rb est H, Rc est H, Cl ou Br, ou alcényle (3 à 5C), et Rd est -NMe-R"', R"' étant alcoyle, hydroxyalcoyle (2 à 4C), ou alcényle (2 à 8C), phénylalcényle, cycloalcoyl(3 à 6C)méthyle, benzyle, benzyle substitué, hétérocyclylméthyle, hétérocyclyléthyle, ou bien R"' est -CH₂CN, -CH₂COOH ou -CORe ou -CH₂CORe pour lesquels soit Re est -OR'e, soit Re est alcoylamino, alcoyl méthyl amino, hétérocyclylamino ou hétérocyclyl méthyl amino,
3) Rb est H, Rd est un radical -NHCH₃ ou -N(CH₃)₂ et Rc est Cl ou Br, ou alcényle (3 à 5C), [si Rd est -N(CH₃)₂],
4) Rb et Rd sont H et Rc est halogène, ou alcoylamino ou dialcoylamino, alcoyloxy, trifluorométhoxy, thioalcoyle, alcoyle (1 à 6C) ou trihalogénométhyle,
5) Rb et Rc sont H et Rd est halogène, ou éthylamino, diéthylamino ou méthyl éthyl amino, alcoyloxy ou trifluorométhoxy, alcoylthio, alcoylsulfinyle, alcoylsulfonyle, alcoyle (1 à 6C), phényle ou trihalogénométhyle,
6) Rb est H et Rc est halogène ou alcoylamino ou dialcoylamino, alcoyloxy ou trifluorométhoxy, thioalcoyle, alcoyle (1 à 3C), et Rd est halogène ou un radical amino, alcoylamino ou dialcoylamino, alcoyloxy ou trifluorométhoxy, thioalcoyle, alcoyle (1 à 6C) ou trihalogénométhyle
7) Rc est H et Rb et Rd sont CH₃

## Claims

1. Group A streptogramin derivative of general formula: 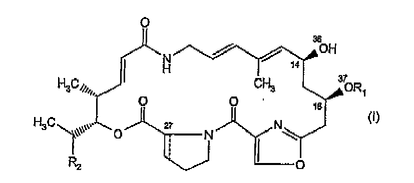 in which:
- R₁ represents an alkyl radical containing 1 to 6 carbons in a straight or branched chain or an alkenyl or alkynyl radical containing 3 to 6 carbons in a straight or branched chain, which radicals may be mono- or polyfluoro radicals, a cycloalkyl radical containing 3 to 6 carbon atoms, a phenylmethyl or heterocyclylmethyl radical in which the heterocyclyl portion is aromatic and represents a pyrrolyl, furyl, thienyl, imidazolyl or pyridyl cycle,
- R₂ represents a hydrogen atom or a methyl or ethyl radical, and
- the bond --- represents a single bond (27R stereochemistry) or a double bond.

2. Streptogramin derivative according to Claim 1,
**characterized in that**
- R₁ represents an alkyl, alkenyl or alkynyl radical,
- R₂ represents a methyl radical, and
- the bond --- represents a single bond (27R stereochemistry) or a double bond.

3. Process for preparing a group A streptogramin derivative according to Claim 1, **characterized in that** a derivative of general formula:
R₁-X (IIa)
for which R₁ is defined as in Claim 1 and X represents a halogen atom or a methylsulphonyloxy, p-toluene-sulphonyloxy or trifluromethylsulphonyloxy radical, is reacted, in the presence of a phase-transfer agent, with a dihydroxy derivative of the streptogramin of general formula: 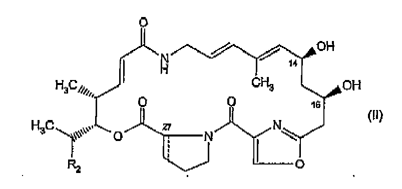 in which R₂ is defined as in Claim 1, the bond --- represents a single bond (27R stereochemistry) or a double bond, and for which derivative the hydroxyl function in position 14 and/or the amide function in position 8 have optionally been protected beforehand, followed, where appropriate, by removal of the protecting radicals.

4. Process for preparing a group A streptogramin derivative according to Claim 1, **characterized in that** the process is performed by desilylation and then 37-O-alkylation of a silyl and 14-O-allyl derivative of general formula: 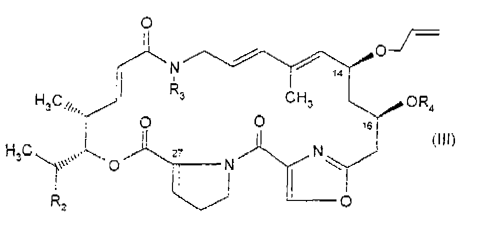 in which R₂ is defined as in Claim 1, the bond --- represents a single bond (27R stereochemistry) or a double bond, and the radical R₃ represents a protecting radical and R₄ represents a silyl radical, by means of a derivative of general formula (IIa) as defined in Claim 2, followed by removal of the allyl radical and the protecting radical R₃ according to the known methods which do not affect the rest of the molecule.

5. Preparation process according to Claim 4 , **characterized in that** the radical R₃ is a t-butoxycarbonyl radical.

6. Group A streptogramin derivative, **characterized in that** it corresponds to the general formula: 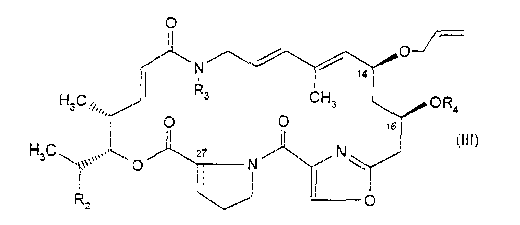 in which the bond ---, R₂, R₃ and R₄ are defined as in Claim 4.

7. Pharmaceutical composition comprising a group A streptogramin derivative according to Claim 1, in pure form or in the form of a combination with at least one group B streptogramin derivative, and/or in the form of a combination with one or more compatible and pharmaceutically acceptable diluents or adjuvants.

8. Pharmaceutical composition according to Claim 7, **characterized in that** the group B streptogramin derivative is chosen from the following natural components: pristinamycin IA, pristinamycin IB, pristinamycin IC, pristinamycin ID, pristinamycin IE, pristinamycin IF, pristinamycin IG, virginiamycin S1, S3 or S4, vernamycin B or C, etamycin or from semisynthetic derivatives of general formula: 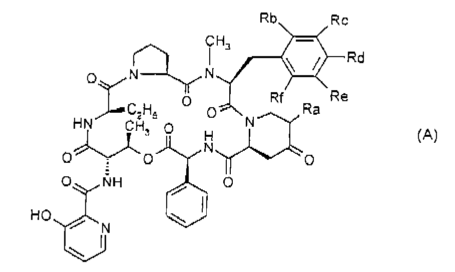 in which:
1) Rb, Rc, Re and Rf are hydrogen atoms, Rd is a hydrogen atom or a dimethylamino radical, and Ra is a radical of structure -CH₂R'a for which R'a is pyrrolidinyl-3-thio, piperidyl-3- (or -4-)thio which may be substituted with alkyl, alkylthio substituted with 1 or 2 hydroxysulphonyl, alkylamino or dialkylamino (which itself may optionally be substituted with mercapto or dialkylamino), or substituted with 1 or 2 piperazine rings, optionally substituted, morpholino, thiomorpholino, piperidino, 1-pyrrolidinyl, 2-, 3- or 4-piperidyl or 2- or 3-pyrrolidinyl rings (which may be substituted with alkyl), or alternatively Ra is a radical of structure =CHR'a for which R'a is pyrrolidinyl-3-amino, piperidyl-3- (or -4-)amino, pyrrolidinyl-3-oxy, piperidyl-3- (or -4-)oxy, pyrrolidinyl-3-thio, piperidyl-3- (or -4-)thio which may be substituted with alkyl, or R'a is alkylamino, alkyloxy or alkylthio which is substituted with 1 or 2 hydroxysulphonyl, alkylamino or dialkylamino (which is itself optionally substituted with dialkylamino), or with trialkylammonio, 4- or 5-imidazolyl, or with 1 or 2 piperazine rings, optionally substituted, morpholino, thiomorpholino, piperidino, 1-pyrrolidinyl, 2-, 3- or 4-piperidyl, or 2- or 3-pyrrolidinyl rings (which may be substituted with alkyl), or Ra is a quinuclidinyl-3- (or -4-)thiomethyl radical, or alternatively
2) Ra is a hydrogen atom and
a) either Rb, Re and Rf are hydrogen atoms, Rd is an
- NHCH₃ or -N(CH₃)₂ radical and Rc is a chlorine or bromine atom, or represents an alkenyl radical containing 3 to 5 carbon atoms [if Rd is -N(CH₃)₂],
b) or Rb, Rd, Re and Rf represent a hydrogen atom and Rc is a halogen or an aminomonoalkyl, aminodialkyl, alkyloxy, trifluoromethyloxy, thioalkyl, C₁ to C₃ alkyl or trihalomethyl radical
c) or Rb, Rc, Re and Rf represent a hydrogen atom and Rd is a halogen or an ethylamino, diethylamino or methylethylamino, alkyloxy or trifluoromethyloxy, thioalkyl, C₁ to C₆ alkyl, aryl or trihalomethyl radical,
d) or Rb, Re and Rf represent a hydrogen atom and Rc is halogen or an aminomonoalkyl or aminodialkyl, alkyloxy or trifluoromethyloxy, thioalkyl or C₁ to C₃ alkyl radical, and Rd is halogen or an amino, aminomonoalkyl or aminodialkyl, alkyloxy or trifluoromethyloxy, thioalkyl, C₁ to C₆ alkyl or trihalomethyl radical,
e) or Rc, Re and Rf represent a hydrogen atom and Rb and Rd represent a methyl radical;
or alternatively from the semisynthetic derivatives of general formula: 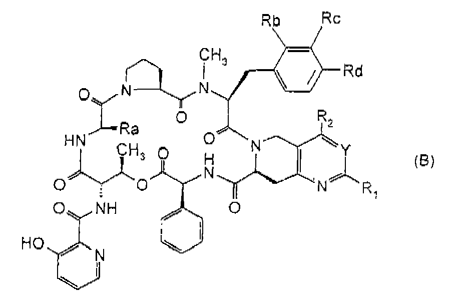 in which:
Y is a nitrogen atom or a radical =CR₃-,
R₁ is a hydrogen atom, an alkyl radical (1 to 8 carbons), alkenyl radical (2 to 8 carbons), cycloalkyl radical (3 to 8 carbons), saturated or unsaturated heterocyclyl radical (3- to 8-membered), phenyl radical, substituted phenyl radical [substituted with one or more halogen atoms or hydroxyl, alkyl, alkyloxy, alkylthio, alkylsulphinyl, alkylsulphonyl, amino, alkylamino or dialkylamino radicals] or a radical NR'R", R' and R", which may be identical or different, possibly being hydrogen atoms or alkyl radicals (1 to 3 carbons) or possibly forming, together with the nitrogen atom to which they are attached, a 3- to 8-membered heterocycle optionally containing another hetero atom chosen from oxygen, sulphur and nitrogen which is optionally substituted [with an alkyl radical, alkenyl radical (2 to 8 carbons), cycloalkyl radical (3 to 6 carbons), saturated or unsaturated heterocyclyl radical (4- to 6-membered), benzyl radical, phenyl radical or substituted phenyl radical as defined above for the definition of R_{1]},
or alternatively, when Y is a radical =CR₃-, R₁ may also be halomethyl, hydroxymethyl, alkyloxymethyl, alkylthiomethyl in which the alkyl portion is optionally substituted with NR'R", alkylsulphinylmethyl, alkylsulphonylmethyl, acyloxymethyl, benzoyloxymethyl, cyclopropylaminomethyl or -(CH₂)ₙNR'R" (n being an integer from 1 to 4 and R' and R" being defined as above), or alternatively, if R₃ is a hydrogen atom, R₁ may also be formyl, carboxyl, alkyloxycarbonyl or -CONR'R" for which R' and R" are defined as above,
or alternatively, when Y is a nitrogen atom, R₁ may also be a radical -XR° for which X is an oxygen or sulphur atom, a sulphinyl or sulphonyl radical, or an NH radical and R° is an alkyl radical (1 to 8 carbons), cycloalkyl radical (3 to 6 carbons), saturated or unsaturated heterocyclyl radical (3- to 8-membered), heterocyclylmethyl radical (3- to 8-membered) in which the heterocyclyl portion is attached to the methyl radical via a carbon atom, phenyl radical, substituted phenyl radical [substituted with one or more halogen atoms or hydroxyl, alkyl, alkyloxy, alkylthio, alkylsulphinyl, alkylsulphonyl, amino, alkylamino or dialkylamino radicals] or a radical -(CH₂)ₙR'R" for which R' and R" are defined as above and n is an integer from 2 to 4, or alternatively, if x represents NH, R° may also represent a hydrogen atom,
R₂ is a hydrogen atom or an alkyl radical (1 to 3 carbons),
R₃ is a hydrogen atom or an alkyl, carboxyl, alkyloxycarbonyl or carbamoyl radical of structure -CO-NR'R" in which R' and R" are defined as above,
Ra is a methyl or ethyl radical, and
Rb, Rc and Rd have the definitions below:
1) Rb and Rc are hydrogen atoms and Rd is a hydrogen atom or a methylamino or dimethylamino radical,
2) Rb is a hydrogen atom, Rc is a hydrogen, chlorine or bromine atom, or represents an alkenyl radical (3 to 5C), and Rd is a radical -NMe-R'" for which R"' represents an alkyl radical, hydroxyalkyl radical (2 to 4C) or alkenyl radical (2 to 8C) optionally substituted with phenyl, cycloalkyl(3 to 6C)methyl radical, benzyl radical, substituted benzyl radical [substituted with one or more halogen atoms or hydroxyl, alkyl, alkyloxy, alkylthio, alkylsulphinyl, alkylsulphonyl, amino, alkylamino or dialkylamino radicals], heterocyclylmethyl radical or heterocyclylethyl radical in which the heterocyclyl portion is saturated or unsaturated and 5- or 6-membered and contains 1 or 2 hetero atoms chosen from sulphur, oxygen and nitrogen which is optionally substituted [with an alkyl radical, alkenyl radical (2 to 8 carbons), cycloalkyl radical (3 to 6 carbons), saturated or unsaturated heterocyclyl radical (4- to 6-membered), phenyl radical, substituted phenyl radical as defined above for the definition of R₁ or benzyl radical], or alternatively R'" represents a cyanomethyl radical or a radical -CH₂CORe for which either Re is -OR'e, R'e being hydrogen, alkyl (1 to 6 carbons), alkenyl (2 to 6 carbons), benzyl or heterocyclylmethyl in which the heterocyclyl portion is 5- or 6-membered and contains 1 or 2 hetero atoms chosen from sulphur, oxygen and nitrogen, or Re is an alkylamino, alkylmethylamino, heterocyclylamino or heteracyclylmethylamino radical in which the heterocyclyl portion is saturated and 5- or 6-membered and contains 1 or 2 hetero atoms chosen from sulphur, oxygen and nitrogen which is optionally substituted with an alkyl, benzyl or alkyloxycarbonyl radical,
3) Rb is a hydrogen atom, Rd is an -NHCH₃ or -N(CH₃)₂ radical and Rc is a chlorine or bromine atom or represents an alkenyl radical (3 to 5C) [if Rd is -N(CH₃)₂],
4) Rb and Rd are hydrogen atoms and Rc is a halogen atom or an alkylamino or dialkylamino, alkyloxy, trifluoromethoxy, thioalkyl, alkyl (1 to 6C) or trihalomethyl radical,
5) Rb and Rc are hydrogen atoms and Rd is a halogen atom or an ethylamino, diethylamino or methylethylamino, alkyloxy or trifluoromethoxy, alkylthio, alkylsulphinyl, alkylsulphonyl, alkyl (1 to 6C), phenyl or trihalomethyl radical,
6) Rb is a hydrogen atom and Rc is a halogen atom or an alkylamino or dialkylamino, alkyloxy or trifluoromethoxy, thioalkyl or alkyl (1 to 3C) radical and Rd is a halogen atom or an amino, alkylamino or dialkylamino, alkyloxy or trifluoromethoxy, thioalkyl, alkyl (1 to 6C) or trihalomethyl radical,
7) Rc is a hydrogen atom and Rb and Rd represent a methyl radical,
as well as the salts thereof,
or alternatively from the semisynthetic derivatives of general formula: 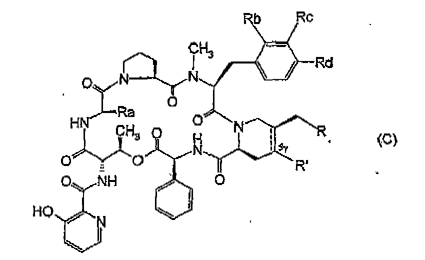
in which R represents a radical -NR₁R₂ or -SR₃ [for which R₁ and R₂, which may be identical or different, represent H, alkyl (1 to 8C) optionally substituted with OH, alkenyl (3 to 8C), cycloalkyl (3 to 8C), alkyloxy (1 to 8C), dialkylamino, phenylalkyl which is optionally substituted [with one or more halogen or alkyl, hydroxyalkyl, alkyloxy or dialkylamino], saturated or unsaturated heterocyclylalkyl (3- to 8-membered) containing one or more hetero atoms chosen from N, S and O, or alternatively R₁ and R₂ form, together with the nitrogen atom, a saturated, partially saturated or unsaturated mono- or polycyclic heterocycle (3- to 12-membered) optionally containing another hetero atom chosen from N, S and O, and optionally substituted [with one or more OH, alkyl, phenyl which is optionally substituted with a halogen atom, phenylalkyl, phenylalkenyl (alkenyl containing 2 to 4C), hydroxyalkyl, acyl, alkyloxycarbonyl or heterocyclyl or heterocyclylcarbonyl in which the heterocyclyl portion is saturated or unsaturated (4- to 6-membered) and contains one or more hetero atoms chosen from N, S and O], R₃ is alkyl (1 to 8C) or cycloalkyl (3 to 8C) substituted with -NR₁R₂ for which R₁ and R₂, which may be identical or different, are H or alkyl or form, together with the nitrogen atom to which they are attached, a heterocycle as defined above, or alternatively R₃ is saturated or unsaturated heterocyclyl or heterocyclylmethyl (3- to 7-membered) optionally containing another hetero atom chosen from oxygen, sulphur and nitrogen and optionally substituted with an alkyl radical; 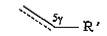 represents the residue of an unsaturated ring which is not substituted in the 5γ position: 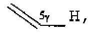 or the residue of a saturated ring which is substituted in the 5γ position with a fluoro radical: 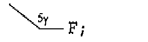 Ra is an Me or Et radical and Rb, Rc and Rd are defined below:
1) Rb and Rc are H and Rd is H or an MeNH or NMe₂ radical,
2) Rb is H, Rc is H, Cl or Br, or alkenyl (3 to 5C), and Rd is -NMe-R"', R'" being alkyl, hydroxyalkyl (2 to 4C) or alkenyl (2 to 8C), phenylalkenyl, cycloalkyl(3 to 6C)methyl, benzyl, substituted benzyl, heterocyclylmethyl or heterocyclylethyl, or alternatively R"' is -CH₂CN, -CH₂COOH or -CORe or -CH₂CORe for which either Re is -OR'e or Re is alkylamino, alkylmethylamino, heterocyclylamino or heterocyclylmethylamino,
3) Rb is H, Rd is an -NHCH₃ or -N(CH₃)₂ radical and Rc is Cl or Br or alkenyl (3 to 5C) [if Rd is -N(CH₃)₂],
4) Rb and Rd are H and Rc is halogen or alkylamino or dialkylamino, alkyloxy, trifluoromethoxy, thioalkyl, alkyl (1 to 6C) or trihalomethyl,
5) Rb and Rc are H and Rd is halogen or ethylamino, diethylamino or methylethylamino, alkyloxy or trifluoromethoxy, alkylthio, alkylsulphinyl, alkylsulphonyl, alkyl (1 to 6C), phenyl or trihalomethyl,
6) Rb is H and Rc is halogen or alkylamino or dialkylamino, alkyloxy or trifluoromethoxy, thioalkyl or alkyl (1 to 3C) and Rd is halogen or an amino, alkylamino or dialkylamino, alkyloxy or trifluoromethoxy, thioalkyl, alkyl (1 to 6C) or trihalomethyl radical,
7) Rc is H and Rb and Rd are CH₃,
as well as the salts thereof, when they exist.

## Patentansprüche

1. Gruppe-A-Streptogramin-Derivat der allgemeinen Formel: 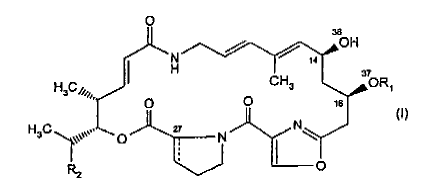 worin:
- R₁ für einen Alkylrest mit 1 bis 6 Kohlenstoffatomen in gerader oder verzweigter Kette oder einen Alkenyl- oder Alkinylrest mit 3 bis 6 Kohlenstoffatomen in gerader oder verzweigter Kette, die mono- oder polyfluoriert sein können, einen Cycloalkylrest mit 3 bis 6 Kohlenstoffatomen, einen Phenylmethylrest oder einen Heterocyclylmethylrest, dessen Heterocyclylteil aromatisch ist und einen Pyrrolyl-, Furyl-, Thienyl-, Imidazolyl- oder Pyridylring darstelllt, steht;
- R₂ für ein Wasserstoffatom oder einen Methyl- oder Ethylrest steht und
- die Bindung --- für eine Einfachbindung (27R-Stereochemie) oder eine Doppelbindung steht.

2. Streptogramin-Derivat nach Anspruch 1, **dadurch gekennzeichnet, daß**
- R₁ für einen Alkyl-, Alkenyl- oder Alkinylrest steht,
- R₂ für einen Methylrest steht und
- die Bindung --- für eine Einfachbindung (27R-Stereochemie) oder eine Doppelbindung steht.

3. Verfahren zur Herstellung eines Gruppe-A-Streptogramin-Derivats nach Anspruch 1, **dadurch gekennzeichnet, daß** man ein Derivat der allgemeinen Formel:
R₁-X (IIa)
worin R₁ die gleiche Bedeutung wie in Anspruch 1 besitzt und X für ein Halogenatom oder einen Methylsulfonyloxy-, p-Toluolsulfonyloxy- oder Trifluormethylsulfonyloxyrest steht, in Gegenwart eines Phasentransferagens mit einem Dihydroxystreptograminderivat der allgemeinen Formel: 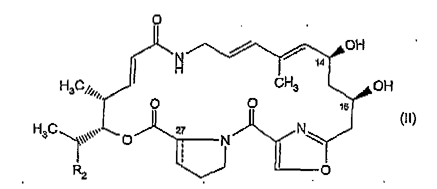 worin R₂ die in Anspruch 1 angegebene Bedeutung besitzt, die Bindung --- für eine Einfachbindung (27R-Stereochemie) oder eine Doppelbindung steht und die Hydroxyfunktion in Position 14 und/oder die Amidfunktion in Position 8 gegebenenfalls vorher geschützt worden sind, umsetzt und danach gegebenenfalls die Schutzgruppe(n) abspaltet.

4. Verfahren zur Herstellung eines Gruppe-A-Streptogramin-Derivats nach Anspruch 1, **dadurch gekennzeichnet, daß** man ein Silyl-14-O-allyl-Derivat der allgemeinen Formel: 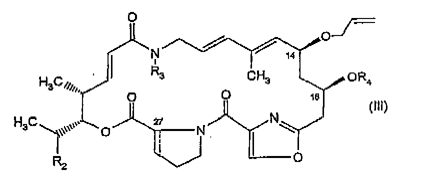 worin R₂ die in Anspruch 1 angegebene Bedeutung besitzt, die Bindung --- für eine Einfachbindung (27R-Stereochemie) oder eine Doppelbindung steht, der Rest R₃ für eine Schutzgruppe steht und R₄ für einen Silylrest steht, mit einem Derivat der allgemeinen Formel (IIa) gemäß Anspruch 2 desilyliert und dann 37-O-alkyliert und danach den Allylrest und die Schutzgruppe R₃ nach bekannten Methoden, die keinen Einfluß auf den Rest des Moleküls haben, abspaltet.

5. Herstellungsverfahren nach Anspruch 4 **dadurch gekennzeichnet, daß** es sich bei dem Rest R₃ um einen t-Butoxycarbonylrest handelt.

6. Gruppe-A-Streptogzamin-Derivat, **dadurch gekennzeichnet, daß** es der folgenden allgemeinen Formel entspricht: 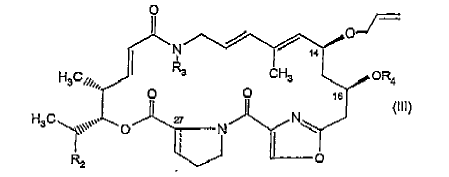 worin die Bindung ---, R₂, R₃ und R₄ die in Anspruch 4 angegebene Bedeutung besitzen.

7. Pharmazeutische Zusammensetzung, enthaltend ein Gruppe-A-Streptogramin-Derivat nach Anspruch 1 in reiner Form oder in Form einer Kombination mit mindestens einem Gruppe-B-Streptogramin-Derivat und/oder in Form einer Kombination mit einem oder mehreren verträglichen und pharmazeutisch unbedenklichen Verdünnungsmitteln oder Hilfsstoffen.

8. Pharmazeutische Zusammensetzung nach Anspruch 7, **dadurch gekennzeichnet, daß** das Gruppe-B-Streptogramin-Derivat unter den natürlichen Komponenten Pristinamycin IA, Pristinamycin IB, Pristinamycin IC, Pristinamycin ID, Pristinamycin IE, Pristinamycin IF, Pristinamycin IG, virginiamycin S1, S3 oder S4, Vernamycin B oder C, Etamycin, den halbsynthetischen Derivaten der allgemeinen Formel: 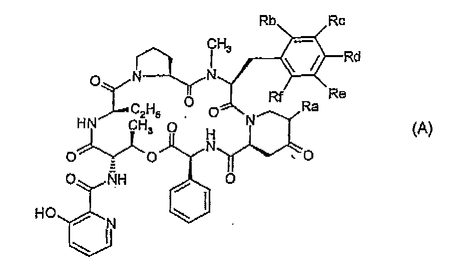 worin
1) Rb, Rc, Re und Rf für Wasserstoffatome stehen, Rd für ein wasserstoffatom oder einen Dimethylaminorest steht und Ra für einen Rest der Struktur -CH₂R'a steht, wobei R'a Pyrrolidinyl-3-thio, Piperidyl-3-thio oder Piperidyl-4-thio, gegebenenfalls substituiert durch Alkyl, Alkylthio, substituiert durch 1 oder 2 Hydroxysulfonyl, Alkylamino, Dialkylamino (selbst gegebenenfalls substituiert durch Mercapto oder Dialkylamino) oder substituiert durch 1 oder 2 gegebenenfalls substituierte Piperazinringe, Morpholino, Thiomorpholino, Piperidino, 1-Pyrrolidinyl, 2-, 3- oder 4-Piperidyl oder 2-oder 3-Pyrrolidinyl (gegebenenfalls substituiert durch Alkyl), bedeutet, oder Ra für einen Rest der Struktur =CH₂R'a steht, wobei R'a Pyrrolidinyl-3-amino, Piperidyl-3-amino oder Piperidyl-4-amino, Pyrrolidinyl-3-oxy, Piperidyl-3-oxy oder Piperidyl-4-oxy, Pyrrolidinyl-3-thio, Piperidyl-3-thio oder Piperidyl-4-thio, gegebenenfalls substituiert durch Alkyl, oder Alkylamino, Alkyloxy oder Alkylthio, substituiert durch 1 oder 2 Hydroxysulfonyl, Alkylamino, Dialkylamino (selbst gegebenenfalls substituiert durch Dialkylamino), oder durch Trialkylammonio, 4- oder 5-Imidazolyl, oder durch 1 oder 2 gegebenenfalls substituierte Piperazinringe, Morpholino, Thiomorpholino, Piperidino, 1-Pyrrolidinyl, 2-, 3- oder 4-Piperidyl oder 2- oder 3-Pyrrolidinyl (gegebenenfalls substituiert durch Alkyl), bedeutet, oder Ra für einen Chinuclidinyl-3-thiomethyl- oder Chinuclidinyl-4-thiomethylrest steht, oder
2) Ra für ein Wasserstoffatom steht und
a) entweder Rb, Re und Rf für ein Wasserstoffatom stehen, Rd für einen -NHCH₃- oder -N(CH₃)₂-Rest steht und Rc für ein Chlor- oder Bromatom oder einen Alkenylrest mit 3 bis 5 Kohlenstoffatomen [wenn Rd für -N(CH₃)₂ steht] steht,
b) oder Rb, Rd, Re und Rf für ein Wasserstoffatom stehen und Rc für ein Halogen oder einen Aminomonoalkyl-, Aminodialkyl-, Alkyloxy-, Trifluormethyloxy-, Thioalkyl-, C₁-C₃-Alkyl- oder Trihalogenmethylrest steht,
c) oder Rb, Rc, Re und Rf für ein wasserstoffatom stehen und Rd für ein Halogen oder einen Ethylamino-, Diethylamino- oder Methylethylamino-, Alkyloxy- oder Trifluormethyloxy-, Thioalkyl-, C₁-C₆-Alkyl- oder Trihalogenmethylrest steht,
d) oder Rb, Re und Rf für ein Wasserstoffatom stehen und Rc für ein Halogen oder einen Aminomonoalkyl- oder Aminodialkyl-, Alkyloxy- oder Trifluormethyloxy-, Thioalkyl- oder C₁-C₃-Alkylrest steht und Rd für ein Halogen oder einen Amino-, Aminomonoalkyl- oder Aminodialkyl-, Alkyloxy- oder Trifluormethyloxy-, Thioalkyl-, C₁-C₆-Alkyl- oder Trihalogenmethylrest steht,
e) oder Rc, Re und Rf für ein Wasserstoffatom stehen und Rb und Rd für einen Methylrest stehen;
den halbsynthetischen Derivaten der allgemeinen Formel: 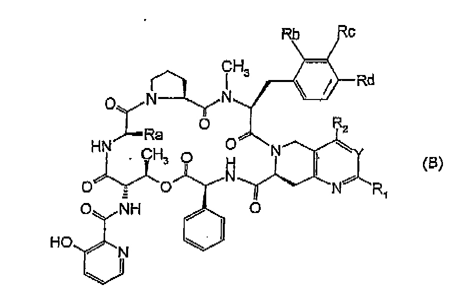 worin
Y für ein Stickstoffatom oder einen =CR₃-Rest steht,
R₁ für ein Wasserstoffatom, einen Alkylrest (1 bis 8 Kohlenstoffatome), einen Alkenylrest (2 bis 8 Kohlenstoffatome), einen Cycloalkylrest (3 bis 8 Kohlenstoffatome), einen gesättigten oder ungesättigten Heterocyclylrest (3- bis 8-gliedrig), einen Phenylrest, einen [durch ein oder mehrere Halogenatome oder Hydroxy-, Alkyl-, Alkyloxy-, Alkylthio-, Alkylsulfinyl-, Alkylsulforiyl-, Amino-, Alkylamino- oder Dialkylaminoreste] substituierten Phenylrest oder einen NR'R" -Rest steht, wobei R' und R" gleich oder verschieden sind und Wasserstoffatome oder Alkylreste (1 bis 3 Kohlenstoffatome) bedeuten oder gemeinsam mit dem Stickstoffatom, an das sie gebunden sind, einen 3- bis 8-gliedrigen Heterocyclus, gegebenenfalls mit einem weiteren unter Sauerstoff, Schwefel und Stickstoff ausgewählten Heteroatom, gegebenenfalls substituiert [durch einen Alkylrest, einen Alkenylrest (2 bis 8 Kohlenstoffatome), einen Cycloalkylrest (3 bis 6 Kohlenstoffatome), einen gesättigten oder ungesättigten Heterocyclylrest (4- bis 6-gliedrig), einen Benzylrest, einen Phenylrest oder einen wie oben für die Definition von R₁ definiert substituierten Phenylrest], bilden,
oder wenn Y für einen =CR₃-Rest steht, R₁ auch für Halogenmethyl, Hydroxymethyl, Alkyloxymethyl, Alkylthiomethyl, dessen Alkylteil gegebenenfalls durch NR'R" substituiert ist, Alkylsulfinylmethyl, Alkylsulfonylmethyl, Acyloxymethyl, Benzoyloxymethyl, Cyclopropylaminomethyl oder -(CH₂)ₙNR'R" (wobei n eine ganze Zahl von 1 bis 4 bedeutet und R' und R" die oben angegebene Bedeutung besitzen), stehen kann, oder wenn R₃ für ein Wasserstoffatom steht, R₁ auch für Formyl, Carboxy, Alkyloxycarbonyl oder -CONR'R", worin R' und R" die oben angegebene Bedeutung besitzen, stehen kann, oder wenn Y für ein Stickstoffatom steht, R₁ auch für einen XR°-Rest stehen kann, wobei X ein Sauerstoff- und Schwefelatom, einen Sulfinyl- oder Sulfonylrest oder einen NH-Rest bedeutet und R° einen Alkylrest (1 bis 8 Kohlenstoffatome), einen Cycloalkylrest (3 bis 6 Kohlenstoffatome), einen gesättigten oder ungesättigten Heterocyclylrest (3- bis 8-gliedrig), einen Heterocyclylmethylrest (3- bis 8-gliedrig), dessen Heterocyclylteil über ein Kohlenstoffatom an den Methylrest gebunden ist, einen Phenylrest, einen [durch ein oder mehrere Halogenatome oder Hydroxy-, Alkyl-, Alkyloxy-, Alkylthio-, Alkylsulfinyl-, Alkylsulfonyl-, Amino-, Alkylamino- oder Dialkylaminoreste] substituierten Phenylrest oder einen -(CH₂)ₙNR'R"-Rest, worin R' und R" die oben angegebene Bedeutung besitzen und n für eine ganze Zahl von 2 bis 4 steht, oder wenn X NH bedeutet, R° auch ein Wasserstoffatom bedeuten kann,
R₂ für ein Wasserstoffatom oder einen Alkylrest (1 bis 3 Kohlenstoffatome) steht,
R₃ für ein Wasserstoffatom oder einen Alkyl-, Carboxy-, Alkyloxycarbonyl- oder Carbamoylrest der Struktur -CO-NR'R", worin R' und R" die oben angegebene Bedeutung besitzen, steht,
Ra für einen Methyl- oder Ethylrest steht und
Rb, Rc und Rd die nachstehenden Bedeutungen besitzen:
1) Rb und Rc stehen für Wasserstoffatome und Rd steht für ein Wasserstoffatom oder einen Methylamino- oder Dimethylaminorest,
2) Rb steht für ein Wasserstoffatom, Rc steht für ein Wasserstoff-, Chlor- oder Bromatom oder einen Alkenylrest (C₃-C₅) und Rd steht für einen -NMe-R"'-Rest, worin R"' einen Alkylrest, einen Hydroxyalkylrest (C₂-C₄) oder einen Alkenylrest (C₂-C₈), gegebenenfalls substituiert durch Phenyl, Cycloalkyl(C₃-C₆)methyl, Benzyl, [durch ein oder mehrere Halogenatome oder Hydroxy-, Alkyl-, Alkyloxy-, Alkylthio-, Alkylsulfinyl-, Alkylsulfonyl-, Amino-, Alkylamino- oder Dialkylaminoreste] substituiertes Benzyl, Heterocyclylmethyl oder Heterocyclylethyl mit gesättigtem oder ungesättigtem und 5- oder 6-gliedrigem Heterocyclylteil mit 1 oder 2 unter Schwefel, Sauerstoff und Stickstoff ausgewählten Heteroatomen, gegebenenfalls substituiert [durch einen Alkylrest, einen Alkenylrest (2 bis 8 Kohlenstoffatome), einen Cycloalkylrest (3 bis 6 Kohlenstoffatome), einen gesättigten oder ungesättigten Heterocyclylrest (4- bis 6-gliedrig), einen Phenylrest oder einen wie oben für die Definition von R₁ definiert substituierten Phenylrest oder Benzyl] bedeutet, oder R"' einen Cyanomethylrest oder einen -CH₂CORe-Rest bedeutet, wobei entweder Re für -OR'e steht, wobei R'e Wasserstoff, Alkyl (1 bis 6 Kohlenstoffatome), Alkenyl (2 bis 6 Kohlenstoffatome), Benzyl oder Heterocyclylmethyl mit 5-oder 6-gliedrigem Heterocyclylteil mit 1 oder 2 unter Schwefel, Sauerstoff und Stickstoff ausgewählten Heteroatomen bedeutet, oder Re für einen Alkylaminorest, einen Alkylmethylaminorest, einen Heterocyclylaminorest oder einen Heterocyclylmethylaminorest mit gesättigtem und 5- oder 6-gliedrigem Heterocyclylteil und 1 bis 2 unter Schwefel, Sauerstoff und Stickstoff ausgewählten Heteroatomen, gegebenenfalls substituiert durch einen Alkyl-, Benzyl- oder Alkyloxycarbonylrest, steht,
3) Rb steht für ein Wasserstoffatom, Rd für einen -NHCH₃- oder -N(CH₃)₂-Rest und Rc steht für ein Chlor- oder Bromatom oder einen Alkenylrest (C₃-C₅) [wenn Rd für -N(CH₃)₂ steht],
4) Rb und Rd stehen für Wassersatoffatome und Rc steht für ein Halogenatom oder einen Alkylamino- oder Dialkylamino-, Alkyloxy-, Trifluormethoxy-, Thioalkyl-, C₁-C₆-Alkyl- oder Trihalogenmethylrest,
5) Rb und Rc stehen für Wasserstoffatome und Rd steht für ein Halogenatom oder einen Ethylamino-, Diethylamino- oder Methylethylamino-, Alkyloxy- oder Trifluormethyloxy-, Alkylthio-, Alkylsulfinyl-, Alkylsulfonyl-, C₁-C₆-Alkyl-, Phenyl- oder Trihalogenmethylreet,
6) Rb steht für ein Wasserstoffatom und Rc steht für ein Halogenatom oder einen Alkylamino- oder Dialkylamino-, Alkyloxy- oder Trifluormethoxy-, Thioalkyl- oder C₁-C₃-Alkylrest und Rd steht für ein Halogenatom oder einen Amino-, Alkylamino- oder Dialkylamino-, Alkyloxy- oder Trifluormethoxy-, Thioalkyl-, C₁-C₆-Alkyl- oder Trihalogenmethylrest,
7) Rc steht für ein Wasserstoffatom und Rb und Rd stehen für einen Methylrest,
sowie deren Salzen,
oder den halbsynthetischen Derivaten der allgemeinen Formel: 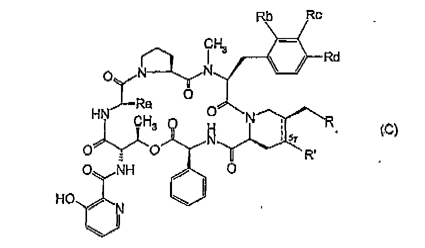 worin R für einen -NR₁R₂- oder -SR₃-Rest steht [wobei R₁ und R₂ gleich oder verschieden sind und H, Alkyl (C₁-C₈), gegebenenfalls substituiert durch OH, Alkenyl (C₃-C₈), Cycloalkyl (C₃-C₈), Alkyloxy (C₁-C₈), Dialkylamino, Phenylalkyl, gegebenenfalls [durch ein oder mehrere Halogen oder Alkyl, Hydroxyalkyl, Alkyloxy oder Dialkylamino] substituiert, gesättigtes oder ungesättigtes Heterocyclylalkyl (3- bis 8-gliedrig) mit 1 oder mehr unter N, S und O ausgewählten Heteroatomen bedeuten oder R₁ und R₂ gemeinsam mit dem Stickstoffatom einen mono- oder polycyclischen, gesättigten, teilweise gesättigten oder ungesättigten Heterocyclus (3- bis 12-gliedrig), gegebenenfalls mit einem weiteren unter N, S oder O und gegebenenfalls [durch ein oder mehrere OH, Alkyl, Phenyl, gegebenenfalls substituiert durch ein Halogenatom, Phenylalkyl, Phenylalkenyl (Alkenyl mit 2 bis 4 Kohlenstoffatomen), Hydroxyalkyl, Acyl, Alkyloxycarbonyl oder Heterocyclyl oder Heterocyclylcarbonyl mit gesättigtem oder ungesättigtem Heterocyclylteil (4- bis 6-gliedrig) mit 1 oder mehr unter N, S und O ausgewählten Heteroatomen] substituiert, bilden, R₃ Alkyl (C₁-C₈) oder Cycloalkyl (C₃-C₈), substituiert durch -NR₁R₂, worin R₁ und R₂ gleich oder verschieden sind und für H oder Alkyl stehen oder gemeinsam mit dem Stickstoffatom, an das sie gebunden sind, einen Heterocyclus gemäß obiger Definition bilden, bedeutet oder R₃ gesättigtes oder ungesättigtes Heterocyclyl oder Heterocyclylmethyl (3- bis 7-gliedrig), gegebenenfalls mit einem weiteren unter Sauerstoff, Schwefel und Stickstoff ausgewählten Heteroatom und gegebenenfalls durch einen Alkylrest substituiert, bedeutet; 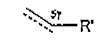 für den Rest eines ungesättigten, in 5γ-Position unsubstituierten Rings: 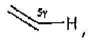 oder für den Rest eines ungesättigten, in 5γ-Position durch einen Fluorrest substituierten Rings : 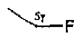 steht;
Ra für einen Me- oder Et-Rest steht und Rb, Rc und
Rd die nachstehenden Bedeutungen besitzen:
1) Rb und Rc stehen für H und Rd steht für H oder einen MeNH- oder NMe₂-Rest,
2) Rb steht für H, Rc steht für H, Cl oder Br oder Alkenyl (C₃-C₅) und Rd steht für -NMe-R"', worin R"' Alkyl, Hydroxyalkyl (C₂-C₄) oder Alkenyl (C₂-C₈), Phenylalkenyl, Cycloalkyl (C₃-C₆) methyl, Benzyl, substituiertes Benzyl, Heterocyclylmethyl, Heterocyclylethyl bedeutet oder R"' -CH₂CN, -CH₂COOH oder -CORe oder -CH₂CORe bedeutet, wobei entweder Re für -OR'e oder Re für Alkylamino, Alkylmethylamino, Heterocyclylamino oder Heterocyclylmethylamino steht,
3) Rb für H steht, Rd steht für einen -NHCH₃- oder -N(CH₃)₂-Rest und Rc steht für Cl oder Br oder Alkenyl (C₃-C₅) [wenn Rd für -N(CH₃)₂ steht],
4) Rb und Rd stehen für H und Rc steht für Halogen oder Alkylamino oder Dialkylamino, Alkyloxy, Trifluormethoxy, Thioalkyl, C₁-C₆-Alkyl oder Trihalogenmethyl,
5) Rb und Rc stehen für H und Rd steht für Halogen oder Ethylamino, Diethylamino oder Methylethylamino, Alkyloxy oder Trifluormethyloxy, Alkylthio, Alkylsulfinyl, Alkylsulfonyl, C₁-C₆-Alkyl, Phenyl oder Trihalogenmethyl,
6) Rb steht für H und Rc steht für Halogen oder Alkylamino oder Dialkylamino, Alkyloxy oder Trifluormethoxy, Thioalkyl oder C₁-C₃-Alkyl und Rd steht für Halogen oder einen Amino-, Alkylamino- oder Dialkylamino-, Alkyloxy- oder Trifluormethoxy-, Thioalkyl-, C₁-C₆-Alkyl- oder Trihalogenmethylrest,
7) Rc steht für H und Rb und Rd stehen für CH₃, sowie deren Salzen, sofern sie existieren,
ausgewählt ist.
